(19) Europäisches Patentamt
European Patent Office
Office européen des brevets

(11) **EP 2 855 694 B1**

(12) **EUROPEAN PATENT SPECIFICATION**

(45) Date of publication and mention
of the grant of the patent:
**26.12.2018 Bulletin 2018/52**

(21) Application number: **13796744.4**

(22) Date of filing: **31.05.2013**

(51) Int Cl.:
*A61B 17/435* (2006.01)    *C12M 1/00* (2006.01)
*C12M 1/36* (2006.01)    *C12M 3/00* (2006.01)
*C12N 5/073* (2010.01)    *C12Q 1/02* (2006.01)
*G01N 21/75* (2006.01)    *G01N 33/50* (2006.01)
*G06K 9/00* (2006.01)    *G06T 7/00* (2017.01)
*G06T 7/60* (2017.01)

(86) International application number:
**PCT/US2013/043639**

(87) International publication number:
**WO 2013/181549 (05.12.2013 Gazette 2013/49)**

(54) **IN VITRO EMBRYO BLASTOCYST PREDICTION METHODS**

IN-VITRO-VERFAHREN ZUR VORHERSAGE VON BLASTOZYSTEN

PROCÉDÉS DE PRÉDICTION DE BLASTOCYSTE EMBRYONNAIRE IN VITRO

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **31.05.2012 US 201261653962 P
12.07.2012 US 201261671060 P**

(43) Date of publication of application:
**08.04.2015 Bulletin 2015/15**

(73) Proprietor: **Progyny, Inc.
Menlo Park, CA 94025 (US)**

(72) Inventors:
• **CHEN KIM, Alice A.
Menlo Park
California 94025 (US)**
• **LOEWKE, Kevin E.
Menlo Park
California 94025 (US)**
• **SURAJ, Vaishali
Menlo Park
California 94025 (US)**

(74) Representative: **CMS Cameron McKenna Nabarro
Olswang LLP
Cannon Place
78 Cannon Street
London EC4N 6AF (GB)**

(56) References cited:
**WO-A1-2012/163363    WO-A2-2012/042228
US-A1- 2010 041 090    US-A1- 2012 095 287
US-B2- 7 963 906**

• **CONNIE C WONG ET AL: "Non-invasive imaging
of human embryos before embryonic genome
activation predicts development to the blastocyst
stage", NATURE BIOTECHNOLOGY, vol. 28, no.
10, 1 October 2010 (2010-10-01), pages 1115-1121,
XP055079709, ISSN: 1087-0156, DOI:
10.1038/nbt.1686**
• **MESEGUER ET AL.: 'The use of morphokinetics
as a predictor of embryo implantation' HUMAN
REPRODUCTION vol. 26, no. 10, 09 August 2011,
pages 2658 - 2671, XP055033123**
• **LEMMEN ET AL.: 'Kinetic markers of human
embryo quality using time-lapse recordings of
IVF/ICSI-fertilized oocytes' REPRODUCTIVE
BIOMEDICINE ONLINE vol. 17, no. 3., 30 July
2008, pages 385 - 391, XP003028429**
• **CHAVEZ ET AL.: 'Dynamic blastomere behaviour
reflects human embryo ploidy by the four-cell
stage' NATURE COMMUNICATION
(NCOMMS2249) 04 December 2012, XP055169858
Retrieved from the Internet:
<URL:http://www.ncbi.nlm.nih.gov/pmc/articl
es/PMC3535341/pdf/ncomms2249.pdf>
[retrieved on 2013-10-07]**

EP 2 855 694 B1

• ANTCZAK ET AL.: 'Temporal and spatial aspects of fragmentation in early human embryos: possible effects on developmental competence and association with the differential elimination of regulatory proteins from polarized domains' HUMAN REPRODUCTION vol. 14, no. 2, 1999, pages 429 - 477, XP055177940
• KIRKEGAARD ET AL.: 'Time-lapse monitoring as a tool for clinical embryo assessment' HUMAN REPRODUCTION vol. 27, no. 5, 14 March 2012, pages 1277 - 1285, XP055177945
• HLINKA ET AL.: 'Time-lapse cleavage rating predicts human embryo viability.' PHYSIOL RES. vol. 61, no. 5, 14 December 2012, pages 513 - 525, XP055079684

**Description**

FIELD OF THE INVENTION

This invention relates to the field of biological and clinical testing, and particularly the imaging and evaluation of zygotes/embryos, oocytes, and stem cells from both humans and animals.

BACKGROUND OF THE INVENTION

Infertility is a common health problem that affects 10-15% of couples of reproductive-age. In the United States alone in the year 2006, approximately 140,000 cycles of in vitro fertilization (IVF) were performed (cdc.gov/art). This resulted in the culture of more than a million embryos annually with variable, and often ill-defined, potential for implantation and development to term. The live birth rate, per cycle, following IVF was just 29%, while on average 30%> of live births resulted in multiple gestations (cdc.gov/art). Multiple gestations have well-documented adverse outcomes for both the mother and fetuses, such as miscarriage, pre-term birth, and low birth rate. Potential causes for failure of IVF are diverse; however, since the introduction of IVF in 1978, one of the major challenges has been to identify the embryos that are most suitable for transfer and most likely to result in term pregnancy.

The understanding in the art of basic embryo development is limited as studies on human embryo biology remain challenging and often exempt from research funding. Consequently, most of the current knowledge of embryo development derives from studies of model organisms. However, while embryos from different species go through similar developmental stages, the timing varies by species. These differences, and many others make it inappropriate to directly extrapolate from one species to another. (Taft, R.E. (2008) Theriogenology 69(1):10-16). The general pathways of human development, as well as the fundamental underlying molecular determinants, are unique to human embryo development. For example, in mice, embryonic transcription is activated approximately 12 hours post-fertilization, concurrent with the first cleavage division, whereas in humans embryonic gene activation (EGA) occurs on day 3, around the 8-cell stage (Bell, C. E., et al. (2008) Mol. Hum. Reprod. 14:691-701; Braude, P., et al. (1988) Nature 332:459-461; Hamatani, T. et al. (2004) Proc. Natl. Acad. Sci. 101:10326-10331; Dobson, T. et al. (2004) Human Molecular Genetics 13(14):1461-1470). In addition, the genes that are modulated in early human development are unique (Dobson, T. et al. (2004) Human Molecular Genetics 13(14):1461-1470). Moreover, in other species such as the mouse, more than 85% of embryos cultured in vitro reach the blastocyst stage, one of the first major landmarks in mammalian development, whereas cultured human embryos have an average blastocyst formation rate of approximately 30-50%, with a high incidence of mosaicism and aberrant phenotypes, such as fragmentation and developmental arrest (Rienzi, L. et al. (2005) Reprod. Biomed. Online 10:669-681; Alikani, M., et al. (2005) Mol. Hum. Reprod. 11:335-344; Keltz, M. D., et al. (2006) Fertil. Steril. 86:321-324; French, D. B., et al. (2009) Fertil. Steril.). In spite of such differences, the majority of studies of preimplantation embryo development derive from model organisms and are difficult to relate to human embryo development (Zemicka-Goetz, M. (2002) Development 129:815-829; Wang, Q., et al. (2004) Dev Cell. 6:133-144; Bell, C. E., et al. (2008) Mol. Hum. Reprod. 14:691-701; Zemicka-Goetz, M. (2006) Curr. Opin. Genet. Dev. 16:406-412; Mtango, N. R., et al. (2008) Int. Rev. Cell. Mol. Biol. 268:223-290).

Traditionally in IVF clinics, human embryo viability has been assessed by simple morphologic observations such as the presence of uniformly-sized, mononucleate blastomeres and the degree of cellular fragmentation (Rijinders PM, Jansen CAM. (1998) Hum Reprod 13:2869-73; Milki AA, et al. (2002) Fertil Steril 77:1191-5). More recently, additional methods such as extended culture of embryos (to the blastocyst stage at day 5) and analysis of chromosomal status via preimplantation genetic diagnosis (PGD) have also been used to assess embryo quality (Milki A, et al. (2000) Fertil Steril 73:126-9; Fragouli E, (2009) Fertil Steril Jun 21 [EPub ahead of print]; El-Toukhy T, et al. (2009) Hum Reprod 6:20; Vanneste E, et al. (2009) Nat Med 15:577-83). However, potential risks of these methods also exist in that they prolong the culture period and disrupt embryo integrity (Manipalviratn S, et al. (2009) Fertil Steril 91:305-15; Mastenbroek S, et al. (2007) N Engl J Med. 357:9-17).

Recently it has been shown that time-lapse imaging can be a useful tool to observe early embryo development. Some methods have used time-lapse imaging to monitor human embryo development following intracytoplasmic sperm injection (ICSI) (Nagy et al. (1994) Human Reproduction. 9(9): 1743-1748; Payne et al. (1997) Human Reproduction. 12:532-541). Polar body extrusion and pro-nuclear formation were analyzed and correlated with good morphology on day 3. However, no parameters were correlated with blastocyst formation or pregnancy outcomes. Other methods have looked at the onset of first cleavage as an indicator to predict the viability of human embryos (Fenwick, et al. (2002) Human Reproduction, 17:407-412; Lundin, et al. (2001) Human Reproduction 16:2652-2657). However, these methods do not recognize the importance of the duration of cytokinesis or time intervals between early divisions.

Other methods have used time-lapse imaging to measure the timing and extent of cell divisions during early embryo development (WO/2007/144001). However, these methods disclose only a basic and general method for time-lapse imaging of bovine embryos, which are substantially different from human embryos in terms of developmental potential, morphological behavior, molecular and epigenetic programs, and timing and parameters surrounding transfer. For example, bovine embryos take substantially longer to implant compared to human embryos (30 days and 9 days, respectively). (Taft, (2008) Theriogenology 69(1): 10-16. Moreover, no specific imaging parameters or time intervals are disclosed that might be predictive of human embryo viability.

More recently, time-lapse imaging has been used to observe human embryo development during the first 24 hours following fertilization (Lemmen et al. (2008) Reproductive BioMedicine Online 17(3):385-391). The synchrony of nuclei after the first division was found to correlate with pregnancy outcomes. However, this work concluded that early first cleavage was not an important predictive parameter, which contradicts previous studies (Fenwick, et al. (2002) Human Reproduction 17:407-412; Lundin, et al. (2001) Human Reproduction 16:2652-2657).

Connie C Wong et al. 'Non-invasive imaging of human embryos before embryonic genome activation predicts development to the blastocyst stage', Nature Biotechnology, vol. 28, no. 10, 1 October 2010, pages 115-1121, discloses studies of preimplantation human embryo development that correlate time-lapse image analysis and gene expression profiling. Meseguer et al, 'The use of morphokinetics as a predictor of embryo implantation', Human Reproduction, vol. 26, no. 10, 9 August 2011, pages 2658-2671, discloses studies aimed at identifying the morphokinetic parameters specific to embryos that were capable of implanting. US7963906 discloses methods, compositions and kits for determining the developmental potential and/or the presence of chromosomal abnormalities of one or more embryos or pluripotent cells.

## SUMMARY OF THE INVENTION

The invention is as claimed in the claims. Methods, compositions and kits for determining the likelihood that one or more embryos or pluripotent cells in one or more embryos will reach the blastocyst stage and/or usable blastocyst stage are provided. These methods, compositions and kits find use in identifying embryos and oocytes in vitro that have a likelihood of reaching the blastocyst stage and/or usable blastocyst stage, i.e. the ability or capacity to develop into a blastocyst, which are thus useful in methods of treating infertility in humans, and the like.

In some aspects disclosed herein, methods are provided for determining the likelihood that an embryo or a pluripotent cell will reach the blastocyst stage and/or usable blastocyst stage. In some aspects determining the likelihood of reaching the blastocyst stage and/or usable blastocyst stage is determined by selecting with high specificity one or more human embryos that is not likely to reach the blastocyst stage, wherein at least about 70%, 75%, 80%), 85%o, 90%), 95% or more or 100% of the human embryos not selected are not likely to reach the blastocyst stage and/or usable blastocyst stage. In such aspects, cellular parameters of an embryo or pluripotent cell are measured to arrive at a cell parameter measurement. The cell parameter is then employed to provide a determination of the likelihood of the embryo or pluripotent cell to reach the blastocyst stage and/or usable blastocyst stage, which determination may be used to guide a clinical course of action. In some embodiments, the cell parameter is a morphological event that is measurable by time-lapse microscopy. In some embodiments, e.g. when an embryo is assayed, the one or more cell parameters is: the duration of a cytokinesis event, e.g. the time interval between cytokinesis 1 and cytokinesis 2; and the time interval between cytokinesis 2 and cytokinesis 3. In some embodiments, the cell parameter is a morphological event that is measurable by time-lapse microscopy. In some embodiments, e.g. when an embryo is assayed, the one or more cell parameters is: the duration of a cytokinesis event, e.g. the time interval between mitotic cell cycle 1 and mitotic cell cycle 2; and the time interval between mitotic cell cycle 2 and mitotic cell cycle 3. In certain embodiments, the duration of cell cycle 1 is also utilized as a cell parameter. In some embodiments, the duration of the first cytokinesis is not measured. In some embodiments, the cell parameter measurement is employed by comparing it to a comparable cell parameter measurement from a reference embryo, and using the result of this comparison to provide a determination of the likelihood of the embryo to reach the blastocyst stage. In some embodiments, the embryo is a human embryo.

In some aspects disclosed herein, methods are provided for ranking embryos or pluripotent cells for their likelihood of reaching the blastocyst stage and/or usable blastocyst stage relative to the other embryos or pluripotent cells in the group. In such embodiments, one or more cellular parameters of the embryos or pluripotent cells in the group is measured to arrive at a cell parameter measurement for each of the embryos or pluripotent cells. The cell parameter measurements are then employed to determine the likelihood of reaching the blastocyst stage and/or usable blastocyst stage for each of the embryos or pluripotent cells in the group relative to one another, which determination may be used to guide a clinical course of action. In some embodiments, the cell parameter is a morphological event that is measurable by time-lapse microscopy. In some embodiments, e.g. when embryos are ranked, the one or more cell parameters are the duration of a cytokinesis event, e.g. the time interval between cytokinesis 1 and cytokinesis 2; and the time interval between cytokinesis 2 and cytokinesis 3. In some embodiments, e.g. when embryos are ranked, the one or more cell parameters are the duration of a mitotic event, e.g. the time interval between mitotic cell cycle 1 and mitotic cell cycle 2; and the time interval between mitotic cell cycle 2 and mitotic cell cycle 3. In certain embodiments, the duration of cell cycle 1 is also measured. In some embodiments, the one or more cell parameter measurements are employed by comparing the cell parameter measurements from each of the embryos or pluripotent cells in the group to one another to determine the likelihood of reaching the blastocyst stage and/or usable blastocyst stage for the embryos or pluripotent cells relative to one another. In some embodiments, the one or more cell parameter measurements are employed by comparing each cell parameter measurement to a cell parameter measurement from a reference embryo or pluripotent cell to determine the likelihood of reaching the blastocyst stage for each embryo or pluripotent cell, and comparing those likelihoods of reaching the blastocyst stage and/or usable blastocyst stage to determine the likelihood of reaching the blastocyst stage and/or usable blastocyst stage of the embryos or pluripotent cells relative to one another.

In some aspects disclosed herein, methods are provided for providing embryos with a likelihood of reaching the blastocyst stage and/or usable blastocyst stage for transfer to a female for assisted reproduction (IVF). In such aspects, one or more embryos is cultured under conditions sufficient for embryo development. One or more cellular parameters is then measured in the one or more embryos to arrive at a cell parameter measurement. The cell parameter measurement is then employed to provide a determination of the likelihood of reaching the blastocyst stage and/or usable blastocyst stage. The one or more embryos that is likely to reach the blastocyst stage and/or usable blastocyst stage is then transferred into a female.

The invention provides methods for selecting embryos with a likelihood of reaching the blastocyst stage for transfer into a female for IVF by culturing one or more embryos under conditions sufficient for embryo development and determining the morphology grade of said embryo. In one embodiment, the morphology grade is based on cell number, symmetry and fragmentation. In one embodiment, the morphology grade is given as a "good", "fair" or "poor" grade. In another aspect of the invention, the morphology grade is given as a letter grade, (i.e. A, B, C, D, F). In still another embodiment, the morphology grade is given as a numerical grade (i.e. 1, 2, 3, 4, etc) In another embodiment one or more cellular parameters is also measure to arrive at a cellular parameter measurement. In one aspect disclosed herein, the cellular parameter is the time interval between cytokinesis 1 and cytokinesis 2 and/or interval between cytokinesis 2 and cytokinesis 3. In the invention, the cellular parameter measurement is the time interval between mitosis 1 and mitosis 2 and the time interval between mitosis 2 and mitosis 3. In a further embodiment, the cellular parameter measurement is used as an adjunct to the morphology grade in selecting an embryo that is likely to reach the blastocyst stage or usable blastocyst stage for transfer into a female, or freezing for later use. In some embodiments, the cellular parameter measurement is used as an adjunct to the morphology grade in de-selecting an embryo that is not likely to reach the blastocyst stage or usable blastocyst stage. In some embodiments, morphology grading and cellular parameter measurements are done sequentially. In other aspects, morphology grading and cellular parameter measurements are done simultaneously.

BRIEF DESCRIPTION OF THE DRAWINGS

The invention is best understood from the following detailed description when read in conjunction with the accompanying drawings. It is emphasized that, according to common practice, the various features of the drawings are not to-scale. On the contrary, the dimensions of the various features are arbitrarily expanded or reduced for clarity. Included in the drawings are the following figures.

Figure 1 describes early embryo divisions.

Figure 2 describes P2 and P3 prediction window time frames.

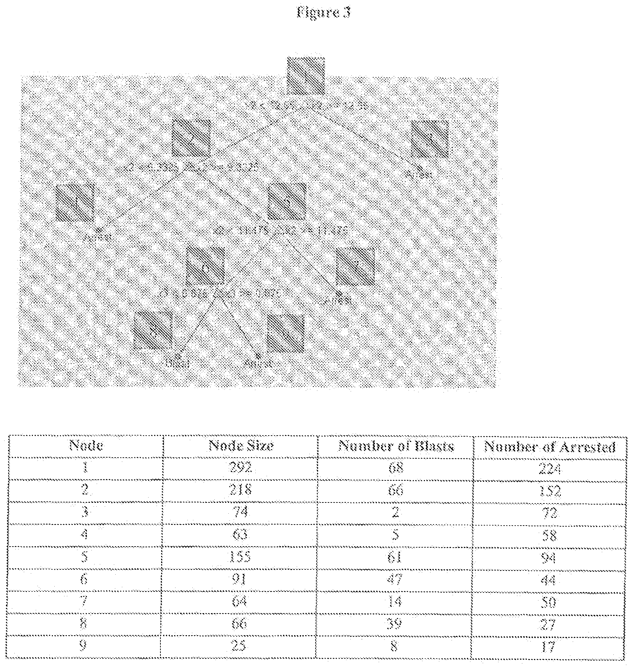

Figure 3 is a data generated by Model 1 for embryo evaluation and a table showing the statistics of the model.

Figure 4 is a data generated by Model 2 for embryo evaluation and a table Showing the statistics of the model.

**Figure 5** is a data generated by Model 3 for embryo evaluation.

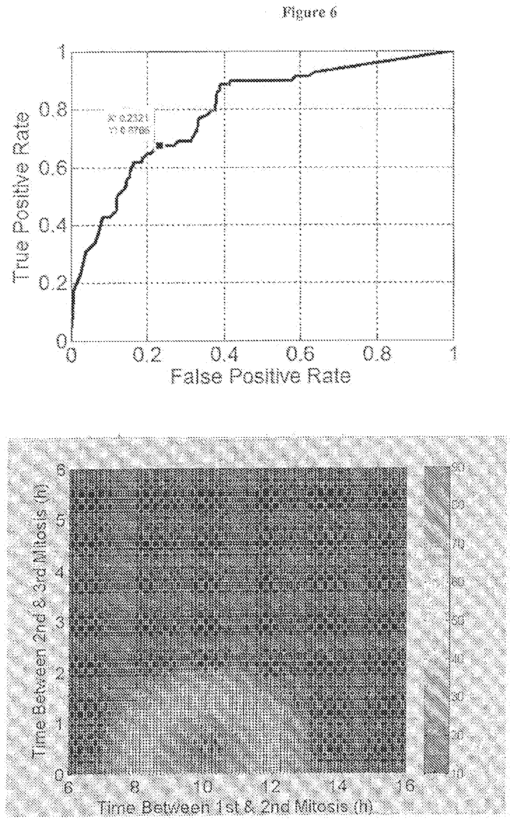

**Figure 6** is a data generated by Model 4 for embryo evaluation.

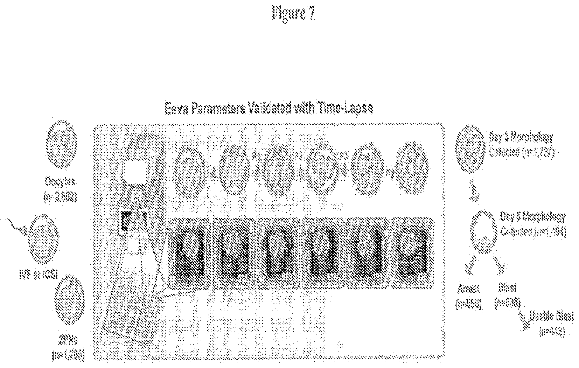

**Figure 7** is a schematic representation of the clinical study workflow at each of five IVF sites. Oocytes were retrieved and fertilized by IVF or ICSI per each clinic's standard protocol. Successfully fertilized 2PNs were cultured in a multiwell dish and imaged in a standard incubator with the **Eeva**$^{TM}$ system, which was set to capture one darkfield image every 5 minutes for 3 days (insets show embryo development and frame numbers from the 1-cell to 8-cell stage). Following imaging, key cell division timing parameters (P1=duration of 1$^{st}$ cytokinesis, P2=time interval between cytokinesis 1 and 2, P3=time interval between cytokinesis 2 and 3) were measured by a panel of expert embryologists and used to develop and independently validate a model which could predict Usable Blastocyst formation by the cleavage stage. Blastocyst formation outcomes and standard morphological criteria were obtained by the study sites.

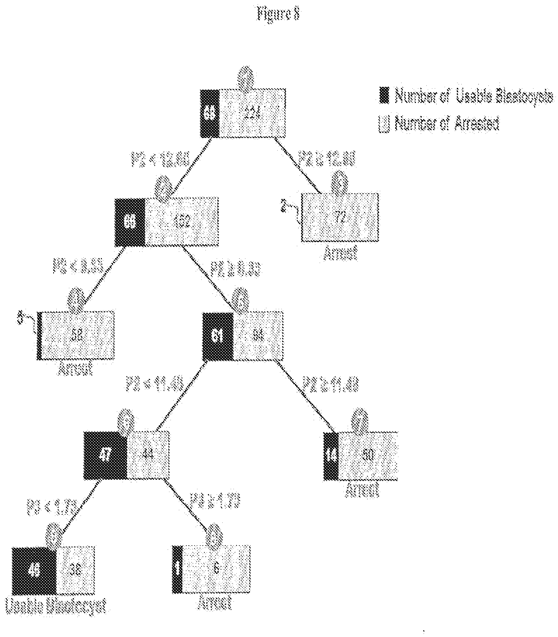

**Figure 8** describes a classification tree for Usable Blastocyst prediction, using 292 embryos cultured to Day 5 or 6 and their Usable Blastocyst (black) or Arrested (grey) outcomes. The classification tree model partitions the data into 10 sub-samples with 5 terminal nodes, based on optimal cell division time periods for P2=time interval between cytokinesis 1 and 2 and P3=time interval between cytokinesis 2 and 3. Usable Blastocyst formation is predicted to be high probability when both P2 and P3 are within specific cell division timing ranges ($9.33 \leq P2 \leq 11.45$ hours and $0 \leq P3 \leq 1.73$ hours), and low probability (likely to Arrest) when either P2 or P3 are outside the specific cell division timing ranges.

**Figure 9** describes cell tracking software developed and validated for enabling image analysis in real-time. Shown are the representative cell tracking results for 1 or 18 human embryos captured at various developmental stages in a single well (left) and a multiwell dish (right). Colored rings represent the cell tracking software's automatic delineation of cell membranes and cell divisions. Using the Eeva software to measure cell divisions and make blastocyst predictions, the overall % agreement compared to manual assessment is 91.0% with 95% CI of 86.0% to 94.3%.

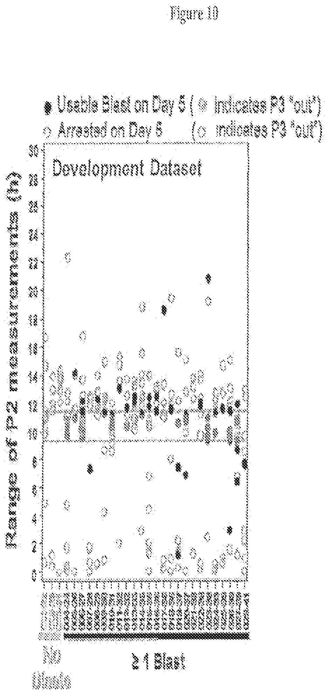

**Figure 10** describes day 5/6 outcomes vs. Eeva predictions for embryo cohorts in the Development Dataset. Each column of datapoints represents a single patient's cohort of embryos and their Day 5/6 Usable Blastocyst (filled circles) or Arrested (open circles) outcomes. Patients are segregated into a group with "No Blasts" or a group with "≥1 Blasts" and ranked by age. The yellow shaded bar highlights all embryos which are within the blastocyst prediction range for P2, with the exception of the blue and red circles. The blue circles are Usable Blastocysts within the P2 range that are out-of-range for P3, and the red circles are Arrested embryos within the P2 range that our out-of-range for P3.

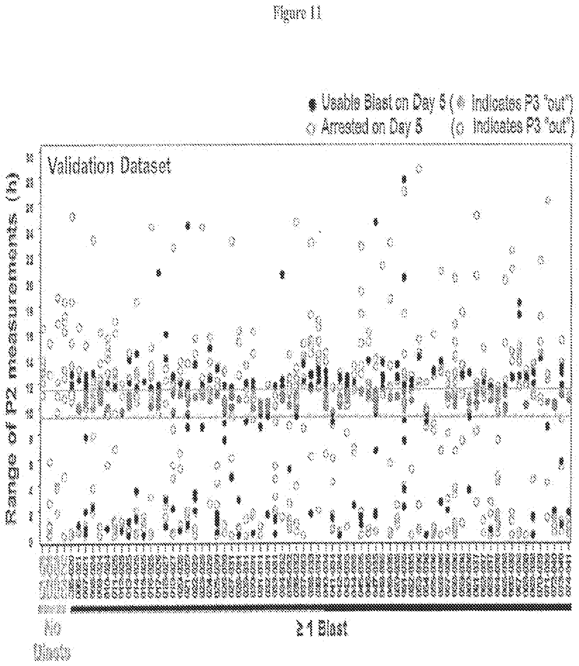

**Figure 11** describes day 5/6 outcomes vs. Eeva predictions for embryo cohorts in the Validation Dataset. Each column of datapoints represents a single patient's cohort of embryos and their Day 5/6 Usable Blastocyst (filled circles) or Arrested (open circles) outcomes. Patients are segregated into a group with "No Blasts" or a group with "≥1 Blasts" and ranked by age. The yellow shaded bar highlights all embryos which are within the blastocyst prediction range for P2, with the exception of the blue and red circles. The blue circles are Usable Blastocysts within the P2 range that are out-of-range for P3, and the red circles are Arrested embryos within the P2 range that our out-of-range for P3.

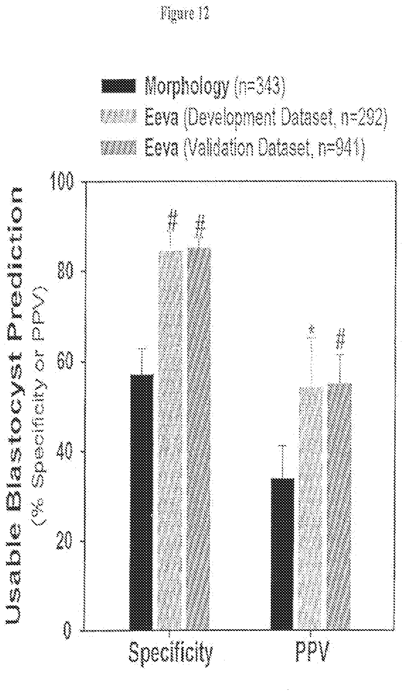

**Figure 12** describes Usable Blastocyst prediction (% Specificity or % PPV) for Morphology on Day 3, compared to Eeva tested on the Development Dataset and Validation Dataset. Error bars represent upper 95% confidence interval. *$p < 0.01$, #$p < 0.0001$.

**Figure 13** describes day 3 embryo selection by individual embryologists (1, 2 and 3) using morphology only versus morphology plus Eeva for (A) all embryos (n=755), and (B) "good morphology" embryos (n=235). "Good morphology" is defined by 6-10 cells, <10% fragmentation and perfect symmetry.

**Figure 14** is a schematic of the "sequential approach" using morphological grading and cellular parameter measurement.

<u>DETAILED DESCRIPTION OF THE INVENTION</u>

Before the present methods and compositions are described, it is to be understood that this invention is not limited to any particular method or composition described, as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limits of that range is also specifically disclosed. Each smaller range between any stated value or intervening value in a stated range and any other stated or intervening value in that stated range is encompassed within the invention. The upper and lower limits of these smaller ranges may independently be included or excluded in the range, and each range where either, neither or both limits are included in the smaller ranges is also encompassed within the invention, subject to any specifically excluded limit in the stated range. Where the stated range includes one or both of the limits, ranges excluding either or both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, some potential and preferred methods and materials are now described. All publications mentioned herein describe the methods and/or materials in connection with which the publications are cited. It is understood that the present disclosure supercedes any disclosure of an incorporated publication to the extent there is a contradiction.

It must be noted that as used herein and in the appended claims, the singular forms "a", "an", and "the" include plural referents unless the context clearly dictates otherwise. Thus, for example, reference to "a cell" includes a plurality of such cells and reference to "the peptide" includes reference to one or more peptides and equivalents thereof, e.g. polypeptides, known to those skilled in the art, and so forth.

The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates which may need to be independently confirmed.

Methods, compositions and kits for determining the likelihood of reaching the blastocyst stage and/or usable blastocyst stage of one or more embryos or pluripotent cells and/or the presence of chromosomal abnormalities in one or more embryos or pluripotent cells are provided. These methods, compositions and kits find use in identifying embryos and oocytes in vitro that are most useful in treating infertility in humans. These and other objects, advantages, and features of the invention will become apparent to those persons skilled in the art upon reading the details of the subject methods and compositions as more fully described below.

The terms "developmental potential' and "developmental competence' are used herein to refer to the ability or capacity of a healthy embryo or pluripotent cell to grow or develop.

The term "specificity" when used herein with respect to prediction and/or evaluation methods is used to refer to the ability to predict or evaluate an embryo for determining the likelihood that the embryo will not develop into a blastocyst by assessing, determining, identifying or selecting embryos that are not likely to reach the blastocyst stage and/or usable blastocyst stage. High specificity as used herein refers to where at least about 70%, 72%, 75%, 77%, 80%, 82%, 85%, 88%, 90%, 92%, 95% or more, or 100% of the human embryos not selected are not likely to reach the blastocyst stage and/or usable blastocyst stage. In some embodiments, embryos that are not likely to reach the blastocyst stage and/or usable blastocyst stage are deselected.

The term "embryo" is used herein to refer both to the zygote that is formed when two haploid gametic cells, e.g. an unfertilized secondary oocyte and a sperm cell, unite to form a diploid totipotent cell, e.g. a fertilized ovum, and to the embryo that results from the immediately subsequent cell divisions, i.e. embryonic cleavage, up through the morula, i.e. 16-cell stage and the blastocyst stage (with differentiated trophoectoderm and inner cell mass).

The term "blastocyst" is used herein to describe all embryos or pluripotent cells that reach cavitation (i.e., the formation of cavities), including those referred to herein as "usable blastocysts".

The term "usable blastocyst" is used herein to refer to any embryo that forms a blastocyst on day 5 and is subsequently either transferred, frozen, or stored by some other means well known by those of skill in the art as part of an in vitro fertilization procedure. Usable blastocysts can also include for example blastocysts with greater potential for developmental competence, greater developmental potential and blastocysts that have the capacity to successfully implant into a uterus. A blastocyst that has the capacity to successfully implant into a uterus has the capacity to go through gestation. A blastocyst that has the capacity to go through gestation has the capacity to be born live. The terms "born live" or "live birth" are used herein to include but are not limited to healthy and/or chromosomally normal (normal number of chromosomes, normal chromosome structure, normal chromosome orientation, etc.) births.

The term "arrested" is used herein to refer to any embryo that does not meet the definition of blastocyst.

The term "pluripotent cell" is used herein to mean any cell that has the ability to differentiate into multiple types of cells in an organism. Examples of pluripotent cells include stem cells, oocytes, and 1-cell embryos (i.e. zygotes).

The term "stem cell" is used herein to refer to a cell or a population of cells which: (a) has the ability to self-renew, and (b) has the potential to give rise to diverse differentiated cell types. Frequently, a stem cell has the potential to give rise to multiple lineages of cells. As used herein, a stem cell may be a totipotent stem cell, e.g. a fertilized oocyte, which gives rise to all of the embryonic and extraembryonic tissues of an organism; a pluripotent stem cell, e.g. an embryonic stem (ES) cell, embryonic germ (EG) cell, or an induced pluripotent stem (iPS) cell, which gives rise to all of embryonic tissues of an organism, i.e. endoderm, mesoderm, and ectoderm lineages; a multipotent stem cell, e.g. a mesenchymal stem cell, which gives rise to at least two of the embryonic tissues of an organism, i.e. at least two of endoderm, mesoderm and ectoderm lineages, or it may be a tissue-specific stem cell, which gives rise to multiple types of differentiated cells of a particular tissue. Tissue-specific stem cells include tissue-specific embryonic cells, which give rise to the cells of a particular tissue, and somatic stem cells, which reside in adult tissues and can give rise to the cells of that tissue, e.g. neural stem cells, which give rise to all of the cells of the central nervous system, satellite cells, which give rise to skeletal muscle, and hematopoietic stem cells, which give rise to all of the cells of the hematopoietic system.

The term "oocyte" is used herein to refer to an unfertilized female germ cell, or gamete. Oocytes of the subject application may be primary oocytes, in which case they are positioned to go through or are going through meiosis I, or secondary oocytes, in which case they are positioned to go through or are going through meiosis II.

By "meiosis" it is meant the cell cycle events that result in the production of gametes. In the first meiotic cell cycle, or meiosis I, a cell's chromosomes are duplicated and partitioned into two daughter cells. These daughter cells then divide in a second meiotic cell cycle, or meiosis II, that is not accompanied by DNA synthesis, resulting in gametes with a haploid number of chromosomes.

By the "germinal vesicle" stage it is meant the stage of a primary oocyte's maturation that correlates with prophase I of the meiosis I cell cycle, i.e. prior to the first division of the nuclear material. Oocytes in this stage are also called "germinal vesicle oocytes", for the characteristically large nucleus, called a germinal vesicle. In a normal human oocyte cultured in vitro, germinal vesicle occurs about 6-24 hours after the start of maturation.

By the "metaphase I" stage it is meant the stage of a primary oocyte's maturation that correlates with metaphase I of the meiosis I cell cycle. In comparison to germinal vesicle oocytes, metaphase I oocytes do not have a large, clearly defined nucleus. In a normal human oocyte cultured in vitro, metaphase I occurs about 12-36 hours after the start of maturation.

By the "metaphase II" stage it is meant the stage of a secondary oocyte's maturation that correlates with metaphase II of the meiosis II cell cycle. Metaphase II is distinguishable by the extrusion of the first polar body. In a normal human oocyte cultured in vitro, metaphase II occurs about 24-48 hours after the start of maturation.

By a "mitotic cell cycle", it is meant the events in a cell that result in the duplication of a cell's chromosomes and the division of those chromosomes and a cell's cytoplasmic matter into two daughter cells. The mitotic cell cycle is divided into two phases: interphase and mitosis. In interphase, the cell grows and replicates its DNA. In mitosis, the cell initiates and completes cell division, first partitioning its nuclear material, and then dividing its cytoplasmic material and its partitioned nuclear material (cytokinesis) into two separate cells.

By a "first mitotic cell cycle" or "cell cycle 1" or "P1" it is meant the time interval from fertilization to the completion of the first cytokinesis event, i.e. the division of the fertilized oocyte into two daughter cells. In instances in which oocytes are fertilized in vitro, the time interval between the injection of human chorionic gonadotropin (HCG) (usually administered prior to oocyte retrieval) to the completion of the first cytokinesis event may be used as a surrogate time interval.

By a "second mitotic cell cycle" or "cell cycle 2" or "P2" it is meant the second cell cycle event observed in an embryo, the time interval between the production of daughter cells from a fertilized oocyte by mitosis and the production of a first set of granddaughter cells from one of those daughter cells (the "leading daughter cell", or daughter cell A) by mitosis. Cell cycle 2 may be measured using several morphological events including the end of cytokinesis land the beginning or end of cytokinesis 2. Upon completion of cell cycle 2, the embryo consists of 3 cells. In other words, cell cycle 2 can be visually identified as the time between the embryo containing 2-cells and the embryo containing 3-cells.

By a "third mitotic cell cycle" or "cell cycle 3" or "P3" it is meant the third cell cycle event observed in an embryo, typically the time interval from the production of a first set of grandaughter cells from a fertilized oocyte by mitosis and the production of a second set of granddaughter cells from the second daughter cell (the "lagging daughter cell" or daughter cell B) by mitosis. Cell cycle 3 may be measured using several morphological events including the end of cytokinesis 2 and the beginning or end of cytokinesis 3. Upon completion of cell cycle 3, the embryo consists of 4 cells. In other words, cell cycle 3 can be visually identified as the time between the embryo containing 3-cells and the embryo containing 4-cells.

By "first cleavage event", it is meant the first division, i.e. the division of the oocyte into two daughter cells, i.e. cell cycle 1. Upon completion of the first cleavage event, the embryo consists of 2 cells.

By "second cleavage event", it is meant the second set of divisions, i.e. the division of leading daughter cell into two granddaughter cells and the division of the lagging daughter cell into two granddaughter cells. In other words, the second cleavage event consists of both cell cycle 2 and cell cycle 3. Upon completion of second cleavage, the embryo consists of 4 cells.

By "third cleavage event", it is meant the third set of divisions, i.e. the divisions of all of the granddaughter cells. Upon completion of the third cleavage event, the embryo typically consists of 8 cells.

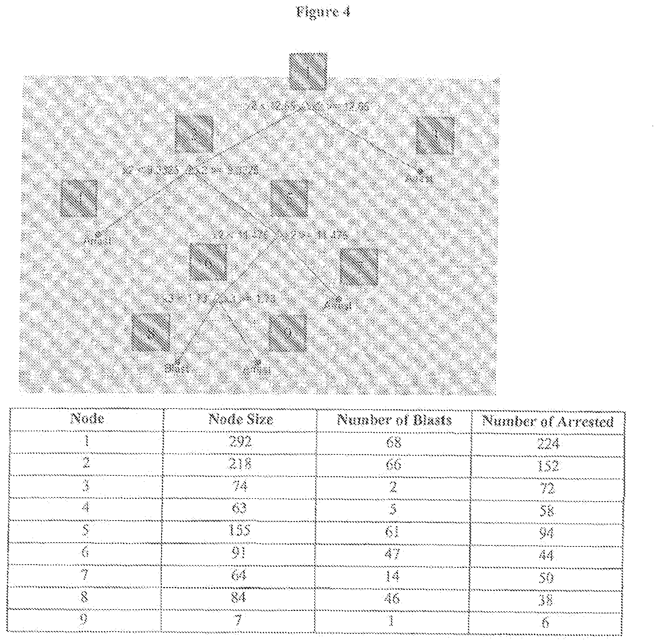

By "cytokinesis" or "cell division" it is meant that phase of mitosis in which a cell undergoes cell division. In other words, it is the stage of mitosis in which a cell's partitioned nuclear material and its cytoplasmic material are divided to produce two daughter cells. The period of cytokinesis is identifiable as the period, or window, of time between when a constriction of the cell membrane (a "cleavage furrow") is first observed and the resolution of that constriction event, i.e. the generation of two daughter cells. The initiation of the cleavage furrow may be visually identified as the point in which the curvature of the cell membrane changes from convex (rounded outward) to concave (curved inward with a dent or indentation). This is illustrated for example in Fig.4 of US Patent No. 7,963,906 top panel by white arrows pointing at 2 cleavage furrows. The onset of cell elongation may also be used to mark the onset of cytokinesis, in which case the period of cytokinesis is defined as the period of time between the onset of cell elongation and the resolution of the cell division.

By "first cytokinesis" or "cytokinesis 1" it is meant the first cell division event after fertilization, i.e. the division of a fertilized oocyte to produce two daughter cells. First cytokinesis usually occurs about one day after fertilization.

By "second cytokinesis" or "cytokinesis 2", it is meant the second cell division event observed in an embryo, i.e. the division of a daughter cell of the fertilized oocyte (the "leading daughter cell", or daughter A) into a first set of two granddaughters.

By "third cytokinesis" or "cytokinesis 3", it is meant the third cell division event observed in an embryo, i.e. the division of the other daughter of the fertilized oocyte (the "lagging daughter cell", or daughter B) into a second set of two granddaughters.

The term "fiduciary marker" or "fiducial marker," is an object used in the field of view of an imaging system which appears in the image produced, for use as a point of reference or a measure. It may be either something placed into or on the imaging subject, or a mark or set of marks in the reticle of an optical instrument.

The term "micro-well" refers to a container that is sized on a cellular scale, preferably to provide for accommodating a single eukaryotic cell.

The term "selecting" or "selection" refers to any method known in the art for moving one or more embryos, blastocysts or other cell or cells as described herein from one location to another location. This can include but is not limited to moving one or more embryos, blastocysts or other cell or cells within a well, dish or other compartment or device so as to separate the selected one or more embryos, blastocysts or other cell or cells disclosed herein from the non-, de- or un-selected one or more embryos, blastocysts or other cell or cells disclosed herein (such as for example moving from one area of a well, dish, compartment or device to another area of a well, dish, compartment or device). This can also include moving one or more embryos, blastocysts or other cell or cells from one well, dish, compartment or device to another well, dish, compartment or device. Any means known in the art for separating or distinguishing the selected one or more embryos, blastocysts or other cell or cells from the non- or un-selected one or more embryos, blastocysts or other cell or cells can be employed with the methods of the present invention. The term "deselection," "deselect" or "deselecting" refers to any method known for moving one or more embryos, blastocysts or other cell or cells as described herein from one location to another location for the purpose of not using them for immediate transfer into a female. For example, an embryo of poor quality may be "deselected" for transfer into a female. The deselected embryos may be transferred to their own compartment, well, dish, device or any other known container and marked for non-transfer. These embryos, may be selected for transfer at later stages if necessary.

In methods disclosed herein, one or more embryos or pluripotent cells is assessed for its likelihood to reach the blastocyst stage and/or usable blastocyst stage by measuring one or more cellular parameters of the embryo(s) or pluripotent cell(s) and employing these measurements to determine the likelihood that the embryo(s) or pluripotent cell(s) will reach the blastocyst stage. Such parameters have been described, for example, in US Patent No. 7,963,906. The information thus derived may be used to guide clinical decisions, e.g. whether or not to transfer an in vitro fertilized embryo, whether or not to transplant a cultured cell or cells.

Examples of embryos that may be assessed by the methods disclosed herein include 1-cell embryos (also referred to as zygotes), 2-cell embryos, 3-cell embryos, 4-cell embryos, 5-cell embryos, 6-cell embryos, 8-cell embryos, etc. typically up to and including 16-cell embryos, morulas, and blastocysts, any of which may be derived by any convenient manner, e.g. from an oocyte that has matured in vivo or from an oocyte that has matured in vitro.

Examples of pluripotent cells that may be assessed by the methods disclosed herein include totipotent stem cells, e.g. oocytes, such as primary oocytes and secondary oocytes; pluripotent stem cells, e.g. ES cells, EG cells, iPS cells, and the like; multipotent cells, e.g. mesenchymal stem cells; and tissue-specific stem cells. They may be from any stage of life, e.g. embryonic, neonatal, a juvenile or adult, and of either sex, i.e. XX or XY.

Embryos and pluripotent cells may be derived from any organism, e.g. any mammalian species, e.g. human, primate, equine, bovine, porcine, canine, feline, etc. Preferable, they are derived from a human. They may be previously frozen, e.g. embryos cryopreserved at the 1-cell stage and then thawed, or frozen and thawed oocytes and stem cells. Alternatively, they may be freshly prepared, e.g., embryos that are freshly prepared from oocytes by in vitro fertilization techniques; oocytes that are freshly harvested and/or freshly matured through in vitro maturation techniques (including, e.g., oocytes that are harvested from in vitro ovarian tissue) or that are derived from pluripotent stem cells differentiated in vitro into germ cells and matured into oocytes; stem cells freshly prepared from the dissociation and culturing of tissues by methods known in the art; and the like. They may be cultured under any convenient conditions known in the art to promote survival,
growth, and/or development of the sample to be assessed, e.g. for embryos, under conditions such as those used in the art of in vitro fertilization; see, e.g., US Patent No. 6,610,543, US Patent No. 6,130,086, US Patent No. 5,837,543; for oocytes, under conditions such as those used in the art to promote oocyte maturation; see, e.g., US Patent No. 5,882,928 and US Patent No. 6,281,013; for stem cells under conditions such as those used in the art to promote maintenance, differentiation, and proliferation, see, e.g. US Patent No. 6,777,233, US Patent No. 7,037,892, US Patent No. 7,029,913, US Patent No. 5,843,780, and US Patent No. 6,200,806, US Application No. 2009/0047263; US Application No. 2009/0068742. Often, the embryos/pluripotent cells are cultured in a commercially available medium such as KnockOut DMEM, DMEM-F12, or Iscoves Modified Dulbecco's Medium that has been supplemented with serum or serum substitute, amino acids, growth factors and hormones tailored to the needs of the particular embryo/pluripotent cell being assessed.

In some embodiments, the embryos/pluripotent cells are assessed by measuring cell parameters by time-lapse imaging. The embryos/pluripotent cells may be cultured in standard culture dishes. Alternatively, the embryos/pluripotent cells may be cultured in custom culture dishes, e.g. custom culture dishes with optical quality micro-wells as described herein. In such custom culture dishes, each micro-well holds a single embryo/pluripotent cell, and the bottom surface of each micro- well has an optical quality finish such that the entire group of embryos within a single dish can be imaged simultaneously by a single miniature microscope with sufficient resolution to follow the cell mitosis processes. The entire group of micro-wells shares the same media drop in the culture dish, and can also include an outer wall positioned around the micro-wells for stabilizing the media drop, as well as fiducial markers placed near the micro-wells. The hydrophobicity of the surface can be adjusted with plasma etching or another treatment to prevent bubbles from forming in the micro-wells when filled with media. Regardless of whether a standard culture dish or a custom culture dish is utilized, during culture, one or more developing embryos may be cultured in the same culture medium, e.g. between 1 and 30 embryos may be cultured per dish.

Images are acquired over time, and are then analyzed to arrive at measurements of the one or more cellular parameters. Time-lapse imaging may be performed with any computer-controlled microscope that is equipped for digital image storage and analysis, for example, inverted microscopes equipped with heated stages and incubation chambers, or custom built miniature microscope arrays that fit inside a conventional incubator. The array of miniature microscopes enables the concurrent culture of multiple dishes of samples in the same incubator, and is scalable to accommodate multiple channels with no limitations on the minimum time interval between successive image capture. Using multiple microscopes eliminates the need to move the sample, which improves the system accuracy and overall system reliability. The individual microscopes in the incubator can be partially or fully isolated, providing each culture dish with its own controlled environment. This allows dishes to be transferred to and from the imaging stations without disturbing the environment of the other samples.

The imaging system for time-lapse imaging may employ brightfield illumination, darkfield illumination, phase contrast, Hoffman modulation contrast, differential interference contrast, polarized light, or fluorescence. In some embodiments, darkfield illumination may be used to provide enhanced image contrast for subsequent feature extraction and image analysis. In addition, red or near-infrared light sources may be used to reduce phototoxicity and improve the contrast ratio between cell membranes and the inner portion of the cells.

Images that are acquired may be stored either on a continuous basis, as in live video, or on an intermittent basis, as in time lapse photography, where a subject is repeatedly imaged in a still picture. Preferably, the time interval between images should be between 1 to 30 minutes in order to capture significant morphological events as described below. In an alternative embodiment, the time interval between images could be varied depending on the amount of cell activity. For example, during active periods images could be taken as often as every few seconds or every minute, while during inactive periods images could be taken every 10 or 15 minutes or longer. Real-time image analysis on the captured images could be used to detect when and how to vary the time intervals. In our methods, the total amount of light received by the samples is estimated to be equivalent to approximately 24 minutes of continuous low-level light exposure for 5-days of imaging. The light intensity for a time-lapse imaging systems is significantly lower than the light intensity typically used on an assisted reproduction microscope due to the low-power of the LEDs (for example, using a 1W LED compared to a typical 100W Halogen bulb) and high sensitivity of the camera sensor. Thus, the total amount of light energy received by an embryo using the time-lapse imaging system is comparable to or less than the amount of energy received during routine handling at an IVF clinic. In addition, exposure time can be significantly shortened to reduce the total amount of light exposure to the embryo/pluripotent cell. For 2-days of imaging, with images captured every 5 minutes at 0.5 seconds of light exposure per image, the total amount of low-level light exposure is less than 5 minutes.

Following image acquisition, the images are extracted and analyzed for different cellular parameters, for example, cell size, thickness of the zona pellucida, degree of fragmentation, symmetry of daughter cells resulting from a cell division, time intervals between the first few mitoses, and duration of cytokinesis.

Cell parameters that may be measured by time-lapse imaging are usually morphological events. For example, in assessing embryos, time-lapse imaging may be used to measure the duration of a cytokinesis event, e.g. cytokinesis 1, cytokinesis 2, cytokinesis 3, or cytokinesis 4, where the duration of a cytokinesis event is defined as the time interval between the first observation of a cleavage furrow (the initiation of cytokinesis) and the resolution of the cleavage furrow into two daughter cells (i.e. the production of two daughter cells). Another parameter of interest is the duration of a cell cycle event, e.g. cell cycle 1, cell cycle 2, cell cycle 3, or cell cycle 4, where the duration of a cell cycle event is defined as the time interval between the production of a cell (for cell cycle 1, the fertilization of an ovum; for later cell cycles, at the resolution of cytokinesis) and the production of two daughter cells from that cell. Other cell parameters of interest that can be measured by time-lapse imaging include time intervals that are defined by these cellular events, e.g. (a) the time interval between cytokinesis 1 and cytokinesis 2, definable as any one of the interval between initiation of cytokinesis 1 and the initiation of cytokinesis 2, the interval between the resolution of cytokinesis 1 and the resolution of cytokinesis 2, the interval between the initiation of cytokinesis 1 and the resolution of cytokinesis 2; or the interval between the resolution of cytokinesis 1 and the initiation of cytokinesis 2; or (b) the time interval between cytokinesis 2 and cytokinesis 3, definable as any one of the interval between the initiation of cytokinesis 2 and the initiation of cytokinesis 3, or the interval between resolution of the cytokinesis 2 and the resolution of cytokinesis 3, or the interval between initiation of cytokinesis 2 and the resolution of cytokinesis 3, or the interval between resolution of cytokinesis 2 and the initiation of cytokinesis 3. In one embodiment, the cellular parameters to be measured consist of the time interval between cytokinesis 1 and cytokinesis 2 and the time interval between cytokinesis 2 and cytokinesis 3.

For the purposes of in vitro fertilization, it is considered advantageous that the embryo be transferred to the uterus early in development, e.g. by day 2 day 3, day 4 or day 5, i.e. up through the 8-cell stage, to reduce embryo loss due to disadvantages of culture conditions relative to the in vitro environment, and to reduce potential adverse outcomes associated with epigenetic errors that may occur during culturing (Katari et al. (2009) Hum Mol Genet. 18(20):3769-78; Sepulveda et al. (2009) Fertil Steril. 91(5):1765-70). Accordingly, it is preferable that the measurement of cellular parameters take place within 2 days of fertilization, although longer periods of analysis, e.g. about 36 hours, about 54 hours, about 60 hours, about 72 hours, about 84 hours, about 96 hours, or more, are also contemplated by the present methods.

Examples of cell parameters in a maturing oocyte that may be assessed by time-lapse imaging include, without limitation, changes in morphology of the oocyte membrane, e.g. oocyte size, the rate and extent of separation from the zona pellucida; changes in the morphology of the oocyte nucleus, e.g. the initiation, completion, and rate of germinal vesicle breakdown (GVBD), presence and location of meiotic spindle and smooth endoplasmic reticulum clustering; the rate and direction of movement of granules in the cytoplasm and nucleus, e.g., ooplasm viscosity and vacuoles changes; the cytokinesis of oocyte and first polar body and the movement of and/or duration of the extrusion of the first polar body. Other parameters include the duration of cytokinesis of the mature secondary oocyte and the second polar body.

Examples of cell parameters in a stem cell or population of stem cells that may be assessed by time-lapse imaging include, without limitation, the duration of cytokinesis events, time between cytokinesis events, size and shape of the stem cells prior to and during cytokinesis events (e.g. changes in morphology and activity as stem cells differentiate including but not limited to elongation, migration, changes in membrane characteristics, changes in nuclear morphology), number of daughter cells produced by a cytokinesis event, spatial orientation of the cleavage furrow, the rate and/or number of asymmetric divisions observed (i.e. where one daughter cell maintains a stem cell while the other differentiates), the rate and/or number of symmetric divisions observed (i.e. where both daughter cells either remain as stem cells or both differentiate), and the time interval between the resolution of a cytokinesis event and when a stem cell begins to differentiate.

Parameters can be measured manually, or they may be measured automatically, e.g. by image analysis software. When image analysis software is employed, image analysis algorithms may be used that employ a probabilistic model estimation technique based on sequential Monte Carlo method, e.g. generating distributions of hypothesized embryo/pluripotent cell models, simulating images based on a simple optical model, and comparing these simulations to the observed image data. When such probabilistic model estimations are employed, cells may be modeled as any appropriate shape, e.g. as collections of ellipses in 2D space, collections of ellipsoids in 3D space, and the like. To deal with occlusions and depth ambiguities, the method can enforce geometrical constraints that correspond to expected physical behavior. To improve robustness, images can be captured at one or more focal planes.

Once cell parameter measurements have been obtained, the measurements are employed to determine the likelihood that the embryo/pluripotent cell will develop into a blastocyst and/or a usable blastocyst.

In some embodiments, the cell parameter measurement is used directly to determine the likelihood that an embryo/pluripotent cell will reach the blastocyst stage. In some embodiments, the cell parameter measurement is used directly to determine the likelihood that an embryo/pluripotent cell will reach the usable blastocyst stage. In other words, the absolute value of the measurement itself is sufficient to determine the likelihood that an embryo/pluripotent cell will reach the blastocyst stage and/or usable blastocyst stage. Examples of this in embodiments using time-lapse imaging to measure cell parameters include, without limitation, the following, which in combination are indicative of the likelihood that an embryo/pluripotent cell will reach the blastocyst stage and/or usable blastocyst stage: (a) a time interval between the resolution of cytokinesis 1 and the onset of cytokinesis 2 that is about 8-15 hours, e.g. about 9-14 hours, about 9-13 hours, about 9-12 hours, or about 9-11.5 hours, or about 9.33-11.45 hours; and (b) a time interval, i.e. synchronicity, between the initiation of cytokinesis 2 and the initiation of cytokinesis 3 that is about 0-6 hours, about 0-5 hours, e.g. about 0-4 hours, about 0-3 hours, about 0-2 hours, or about 0-1.75 hours, or about 0-1.73 hours. In some embodiments, determining the likelihood that the embryo/pluripotent cell will reach the blastocyst stage and/or usable blastocyst stage can additionally include measuring cell parameters, including but not limited to: a cell cycle 1 that lasts about 20-27 hours, e.g. about 25-27 hours. Examples of direct measurements, any of which alone or in combination are indicative of the likelihood that an embryo/pluripotent cell will not reach the blastocyst stage and/or usable blastocyst stage, include without limitation: (a) a time interval between the resolution of cytokinesis 1 and the onset of cytokinesis 2 that lasts more that 15 hour, e.g. about 16, 17, 18, 19, or 20 or more hours, or less than 8 hours, e.g. about 7, 5, 4, or 3 or fewer hours; or (b) a time interval between the initiation of cytokinesis 2 and the initiation of cytokinesis 3 that is 6, 7, 8, 9, or 10 or more hours. In some embodiments, determining the likelihood that the embryo/pluripotent cell will not reach the blastocyst stage and/or usable blastocyst stage can include additionally measuring cell parameters, including but not limited to: a cell cycle 1 that lasts longer than about 27 hours, e.g. 28, 29, or 30 or more hours. In some embodiments, the duration of the first cytokinesis is not measured.

In some embodiments, the cell parameter measurement is employed by comparing it to a cell parameter measurement from a reference, or control, embryo/pluripotent cell, and using the result of this comparison to provide a determination of the likelihood of the embryo/pluripotent cell to reach or not reach the blastocyst stage and/or usable blastocyst stage. The terms "reference" and "control" as used herein mean a standardized embryo or cell to be used to interpret the cell parameter measurements of a given embryo/pluripotent cell and assign a determination of the likelihood of the embryo/pluripotent cell to reach or not reach the blastocyst stage and/or usable blastocyst stage. The reference or control may be an embryo/pluripotent cell that is known to have a desired phenotype, e.g., likely to reach the blastocyst stage and/or usable blastocyst stage, and therefore may be a positive reference or control embryo/pluripotent cell. Alternatively, the reference/control embryo/pluripotent cell may be an embryo/pluripotent cell known to not have the desired phenotype, and therefore be a negative reference/control embryo/pluripotent cell.

In certain embodiments, the obtained cell parameter measurement(s) is compared to a comparable cell parameter measurement(s) from a single reference/control embryo/pluripotent cell to obtain information regarding the phenotype of the embryo/cell being assayed. In yet other embodiments, the obtained cell parameter measurement(s) is compared to the comparable cell parameter measurement(s) from two or more different reference/control embryos or pluripotent cells to obtain more in depth information regarding the phenotype of the assayed embryo/cell. For example, the obtained cell parameter measurements from the embryo(s) or pluripotent cell(s) being assessed may be compared to both a positive and negative embryo or pluripotent cell to obtain confirmed information regarding whether the embryo/cell has the phenotype of interest.

As an example, the resolution of cytokinesis 1 and the onset of cytokinesis 2 in normal human embryos is about 8-15 hours, more often about 9-13 hours, with an average value of about 11 +/- 2.1 hours; i.e. 6, 7, or 8 hours, more usually about 9, 10, 11, 12, 13, 14 or up to about 15 hours. A longer or shorter cell cycle 2 in the embryo being assessed as compared to that observed for a normal reference embryo is indicative of the likelihood that the embryo/pluripotent cell will not reach the blastocyst stage and/or usable blastocyst stage. As a second example, the time interval between the initiation of cytokinesis 2 and the initiation of cytokinesis 3, i.e. the synchronicity of the second and third mitosis, in normal human embryos is usually about 0-5 hours, more usually about 0, 1, 2 or 3 hours, with an average time of about 1 +/- 1.6 hours; a longer interval between the completion of cytokinesis 2 and cytokinesis 3 in the embryo being assessed as compared to that observed in a normal reference embryo is indicative of the likelihood that the embryo/pluripotent cell will not reach the blastocyst stage and/or usable blastocyst stage. As a third example, cell cycle 1 in a normal embryo, i.e. from the time of fertilization to the completion of cytokinesis 1, is typically completed in about 20-27 hours, more usually in about 25-27 hours, i.e. about 15, 16, 17, 18, or 19 hours, more usually about 20, 21, 22, 23, or 24 hours, and more usually about 25, 26 or 27 hours. A cell cycle 1 that is longer in the embryo being assessed as compared to that observed for a normal reference embryo is indicative of the likelihood that the embryo/pluripotent cell will not reach the blastocyst stage and/or usable blastocyst stage. Examples may be derived from empirical data, e.g. by observing one or more reference embryos or pluripotent cells alongside the embryo/pluripotent cell to be assessed. Any reference embryo/pluripotent cell may be employed, e.g. a normal reference that is likely to reach the blastocyst stage and/or usable blastocyst stage, or an abnormal reference sample that is not likely to reach the blastocyst stage. In some cases, more than one reference sample may be employed, e.g. both a normal reference sample and an abnormal reference sample may be used.

In some embodiments, it may be desirable to use cell parameter measurements that are arrived at by time-lapse microscopy.

As discussed above, one or more parameters may be measured and employed to determine the likelihood of reaching the blastocyst stage for an embryo or pluripotent cell. In some embodiments, a measurement of two parameters may be sufficient to arrive at a determination of the likelihood of reaching the blastocyst stage and/or usable blastocyst stage. In some embodiments, it may be desirable to employ measurements of more than two parameters, for example, 3 cell parameters or 4 or more cell parameters.

In certain embodiments, assaying for multiple parameters may be desirable as assaying for multiple parameters may provide for greater sensitivity and specificity. By sensitivity it is meant the proportion of actual positives which are correctly identified as being such. This may be depicted mathematically as:

$$\text{Sensitivity} = \frac{(\text{Number of true positives})}{(\text{Number of true positives} + \text{Number of false negatives})}$$

Thus, in a method in which "positives" are the embryos that have good developmental potential, i.e. that will develop into blastocysts or usable blastocysts, and "negatives" are the embryos that have poor developmental potential, i.e. that will not develop into blastocysts or usable blastocysts, a sensitivity of 100% means that the test recognizes all embryos that will develop into blastocysts or usable blastocysts as such. In some embodiments, the sensitivity of the assay may be about 70%, 80%, 90%, 95%, 98% or more, e.g. 100%. By specificity it is meant the proportion of "negatives" which are correctly identified as such. As discussed above, the term "specificity" when used herein with respect to prediction and/or evaluation methods is used to refer to the ability to predict or evaluate an embryo for determining the likelihood that the embryo will not develop into a blastocyst or usable blastocyst by assessing, determining, identifying or selecting embryos that are not likely to reach the blastocyst stage and/or usable blastocyst stage. This may be depicted mathematically as:

$$\text{Specificity} = \frac{\text{(Number of true negatives)}}{\text{(Number of true negatives + Number of false positives)}}$$

Thus, in a method in which positives are the embryos that are likely to reach the blastocyst stage and/or usable blastocyst stage (i.e., that are likely to develop into blastocysts), and negatives are the embryos that are likely not to reach the blastocyst stage (i.e., that are not likely to develop into blastocysts) a specificity of 100% means that the test recognizes all embryos that will not develop into blastocysts, i.e. will arrest prior to the blastocyst stage. In some embodiments, the specificity can be a "high specificity" of 70%, 72%, 75%, 77%, 80%, 82%, 85%, 88%, 90%, 92%, 95%, 98% or more, e.g. 100%. As demonstrated in the examples sections below, the use of two parameters provides sensitivity of 40%, 57%, 68%, 62%, 68% and specificity of 86%, 88%, 83%, 83%, 77%, respectively. In other words, in one exemplary embodiment, the methods of the invention are able to correctly identify the number of embryos that are going to develop into blastocysts at least about 40%-68% of the time (sensitivity), and the number of embryos that are going to arrest before the blastocyst stage at least about 77%-88% of the time (specificity), regardless of the algorithm model employed, and as such the present invention provides a high specificity method for identifying the embryos that will arrest before the blastocyst stage and not develop into blastocysts. In addition, the specified mean values and/or cut-off points may be modified depending upon the data set used to calculate these values as well as the specific application.

In some embodiments, the measurement of cellular parameters may be used as an adjunct to morphological grading. For example, embryos may be graded at day 1, day 2, day 3, day 4 and/or day 5 for cell number, cell size, symmetry of the blastomeres, cell shape, pronuclear formation, pronuclear number, mutlinucleation, embryo size, degree of compaction, degree of expansion and/or fragmententaion In one embodiment, the presence or absence of fragmentation is measured. In another embodiment, the degree, volume or pattern of fragmentation is measured. In still another embodiment, the percentage of fragmentation is measured. In a particular embodiment, embryos are graded at day 3 for cell number, percentage of fragmentation and symmetry of the blastomeres. Based on these morphological parameters, embryos are graded as "good" or "fair" or "poor" In one embodiment, embryos are determined to be "good" quality embryos by morphological grading when they contain 6-10 cells, have less than about 10% fragmentation and perfect symmetry. In another embodiment, embryos are determined to be "good" quality embryos by morphological grading when they have 7-8 cells, less than 10% fragmentation and perfect symmetry. Conversely, an embryo is determined to be of "poor" quality by morphological assessment when it has less than 6 or greater than 10 cells at day 3, for example, less than 7 or greater than 8 cells, has more than about 10% fragmentation and/or has asymetral blastomeres. An embryo is determined to be of "fair" quality when it falls between the definition of "good" and "poor." For example, when the embryo has 6-10 cells and less than 10% fragmention but less than perfectly symmetrical blastomeres. Day 3 morphological grading is well known in the art and can vary by embryologist. The Instanbul Consensus Workshop on Embryo Assessment: Proceedings of an expert meeting, published in 2011 in Volume 22 of Reproductive Biomedicine Online provides a comprehensive discussion of the state of the art with respect to Day 3 morphological grading. Other similar reviews have been published by Montag, et al. (2011); Desai, et al. (2000); and Machtinger and Racowsky (2013). Furthermore, the variability in morphological grade between embryologists that is a hallmark of morphological grading and which the current invention helps in remedying is discussed extensively in Paternot, et al. (2009). All of these documents are herein specifically and completely incorporated by reference in their entireties. Therefore, one of skill in the art would understand that any day 3 morphological grading may be used with the methods of the current invention.

In a particular embodiment, cellular parameter measurements are used as an adjunct to traditional morphology by concurrently analyzing both cellular parameters and morphology. For example, in an embryo that is determined to be "good" by morphological assessment, an embryologist will determined whether the "good" embryo is also deemed to be "good" by cellular parameter measurement (i.e. have an interval between cytokinesis 1 and cytokinesis that is about 8-15 hours, for example, about $11 \pm 2.1$ hours and/or an interval between cytokinesis 2 and cytokinesis 3 that is less than about 3 hours, for example, about $1 \pm 1.6$ hours). In such instances where both morphological assessment and cellular parameter measurement assessment determine that the embryo is "good," the embryo will be selected to implant into the female recipient or to be frozen for future implantation. Similarly, where both morphological assessment and cellular parameter measurement determine embryo to be of "poor" quality, that embryo should be deselected for non-transfer into a female. Where morphological assessment shows an embryo to be "good" quality and cellular parameter measurement assessment shows the embryo to be "poor" quality, the embryo should not be selected for implantation into a female, but rather should be deselected, or frozen for further analysis should no better quality embryos be found (i.e. embryos determined to have "good" quality by both morphological assessment and cellular parameter measurement assessment). Where an embryo is determined to be of "poor" quality by morphological grading but "good" quality by cellular parameter measurement assessment, the embryo should not be selected or should be deselected for non-transfer into a female or frozen for further analysis should no better quality embryos be found (i.e. embryos determined to have "good" quality by both morphological assessment and cellular parameter measurement assessment).

Alternatively, morphological assessment and cellular parameter measurement assessment can be done sequentially. For example, an embryologist will determine whether or not the embryo is of "good" quality or "poor" quality by morphological assessment at day 3. If the embryo is of "poor" morphological assessment, the embryo will be deselected and no further cellular parameter testing will be done. Conversely, if the embryo is determined to have "good" quality by day 3 morphological assessment, the embryo will be further analyzed to determine the interval between cytokinesis 1 and cytokinesis 2 and/or the interval between cytokinesis 2 and cytokinesis 3 to determine if the embryo is of "good" or "poor" quality by cellular parameter measurement assessment. If the cellular parameter measurement assessment determines the embryo is of "good" quality, that embryo will be selected for transfer into a female or frozen for later transfer. Conversely, if the embryo is determined to have "poor" quality by cellular parameter measurement assessment, that embryo is not selected for transfer or is deselected or is frozen for further evaluation should no better quality embryos be found.

In some embodiments, the assessment of an embryo or pluripotent cell includes generating a written report that includes the artisan's assessment of the subject embryo/pluripotent cell, e.g. "assessment/selection/determination of embryos likely and/or not likely to reach the blastocyst stage and/or usable blastocyst stage", an "assessment of chromosomal abnormalities", etc. Thus, a subject method may further include a step of generating or outputting a report providing the results of such an assessment, which report can be provided in the form of an electronic medium (e.g., an electronic display on a computer monitor), or in the form of a tangible medium (e.g., a report printed on paper or other tangible medium).

A "report," as described herein, is an electronic or tangible document which includes report elements that provide information of interest relating to an assessment arrived at by methods of the invention. A subject report can be completely or partially electronically generated. A subject report includes at least an assessment of the likelihood of the subject embryo or pluripotent cell to reach the blastocyst stage and/or usable blastocyst stage, an assessment of the probability of the existence of chromosomal abnormalities, etc. A subject report can further include one or more of: 1) information regarding the testing facility; 2) service provider information; 3) subject data; 4) sample data; 5) a detailed assessment report section, providing information relating to how the assessment was arrived at, e.g. a) cell parameter measurements taken, b) reference values employed, if any; and 6) other features.

The report may include information about the testing facility, which information is relevant to the hospital, clinic, or laboratory in which sample gathering and/or data generation was conducted. Sample gathering can include how the sample was generated, e.g. how it was harvested from a subject, and/or how it was cultured etc. Data generation can include how images were acquired or gene expression profiles were analyzed. This information can include one or more details relating to, for example, the name and location of the testing facility, the identity of the lab technician who conducted the assay and/or who entered the input data, the date and time the assay was conducted and/or analyzed, the location where the sample and/or result data is stored, the lot number of the reagents (e.g., kit, etc.) used in the assay, and the like. Report fields with this information can generally be populated using information provided by the user.

The report may include information about the service provider, which may be located outside the healthcare facility at which the user is located, or within the healthcare facility. Examples of such information can include the name and location of the service provider, the name of the reviewer, and where necessary or desired the name of the individual who conducted sample preparation and/or data generation. Report fields with this information can generally be populated using data entered by the user, which can be selected from among pre-scripted selections (e.g., using a drop-down menu). Other service provider information in the report can include contact information for technical information about the result and/or about the interpretive report.

The report may include a subject data section, including medical history of subjects from which oocytes or pluripotent cells were harvested, patient age, in vitro fertilization cycle characteristics (e.g. fertilization rate, day 3 follicle stimulating hormone (FSH) level), and, when oocytes are harvested, zygote/embryo cohort parameters (e.g. total number of embryos). This subject data may be integrated to improve embryo assessment and/or help determine the optimal number of embryos to transfer. The report may also include administrative subject data (that is, data that are not essential to the assessment of the likelihood of reaching the blastocyst stage) such as information to identify the subject (e.g., name, subject date of birth (DOB), gender, mailing and/or residence address, medical record number (MRN), room and/or bed number in a healthcare facility), insurance information, and the like), the name of the subject's physician or other health professional who ordered the assessment of developmental potential and, if different from the ordering physician, the name of a staff physician who is responsible for the subject's care (e.g., primary care physician).

The report may include a sample data section, which may provide information about the biological sample analyzed in the assessment, such as the type of sample (embryo or pluripotent cell, and type of pluripotent cell), how the sample was handled (e.g. storage temperature, preparatory protocols) and the date and time collected. Report fields with this information can generally be populated using data entered by the user, some of which may be provided as pre-scripted selections (e.g., using a drop-down menu).

The report may include an assessment report section, which may include information relating to how the assessments/determinations were arrived at as described herein. The interpretive report can include, for example, time-lapse images of the embryo or pluripotent cell being assessed, and/or gene expression results. The assessment portion of the report can optionally also include a recommendation(s) section. For example, where the results indicate that the embryo is likely to reach the blastocyst stage and/or usable blastocyst stage, the recommendation can include a recommendation that a limited number of embryos be transplanted into the uterus during fertility treatment as recommended in the art.

It will also be readily appreciated that the reports can include additional elements or modified elements. For example, where electronic, the report can contain hyperlinks which point to internal or external databases which provide more detailed information about selected elements of the report. For example, the patient data element of the report can include a hyperlink to an electronic patient record, or a site for accessing such a patient record, which patient record is maintained in a confidential database. This latter embodiment may be of interest in an in-hospital system or in-clinic setting. When in electronic format, the report is recorded on a suitable physical medium, such as a computer readable medium, e.g., in a computer memory, zip drive, CD, DVD, etc.

It will be readily appreciated that the report can include all or some of the elements above, with the proviso that the report generally includes at least the elements sufficient to provide the analysis requested by the user (e.g., an assessment of the likelihood of reaching the blastocyst stage).

As discussed above, methods disclosed herein may be used to assess embryos or pluripotent cells to determine the likelihood of the embryos or pluripotent cells to reach the blastocyst stage and/or usable blastocyst stage. This determination of the likelihood of the embryos or pluripotent cells to reach the blastocyst stage and/or usable blastocyst stage may be used to guide clinical decisions and/or actions. For example, in order to increase pregnancy rates, clinicians often transfer multiple embryos into patients, potentially resulting in multiple pregnancies that pose health risks to both the mother and fetuses. Using results obtained from the methods of the invention, the likelihood of reaching the blastocyst stage and/or usable blastocyst stage can be determined for embryos being transferred. As the embryos or pluripotent cells that are likely to reach the blastocyst stage and/or usable blastocyst stage are more likely to develop into fetuses, the determination of the likelihood of the embryo to reach the blastocyst stage and/or usable blastocyst stage prior to transplantation allows the practitioner to decide how many embryos to transfer so as to maximize the chance of success of a full term pregnancy while minimizing risk.

Assessments made by following methods disclosed herein may also find use in ranking embryos or pluripotent cells in a group of embryos or pluripotent cells for their likelihood that the embryos or pluripotent cells will reach the blastocyst stage as well as for the quality of the blastocyst that will be achieved (e.g., in some embodiments this would include the likelihood of reaching the usable blastocyst stage). For example, in some instances, multiple embryos may be capable of developing into blastocysts, i.e. multiple embryos are likely to reach the blastocyst stage. However, some embryos will be more likely to achieve the blastocyst stage, i.e. they will have better likelihood to reach the blastocyst stage, or better likelihood to reach the usable blastocyst stage than other embryos. In some embodiments, some embryos will be likely to achieve the usable blastocyst stage. In such cases, methods disclosed herein may be used to rank the embryos in the group. In such methods, one or more cell parameters for each embryo/pluripotent cell is measured to arrive at a cell parameter measurement for each embryo/pluripotent cell. The one or more cell parameter measurements from each of the embryos or pluripotent cells are then employed to determine the likelihood of the embryos or pluripotent cells relative to one another to reach the blastocyst stage and/or to be a usable blastocyst. In some embodiments, the cell parameter measurements from each of the embryos or pluripotent cells are employed by comparing them directly to one another to determine the likelihood of reaching the blastocyst stage and/or usable blastocyst stage. In some embodiments, the cell parameter measurements from each of the embryos or pluripotent cells are employed by comparing the cell parameter measurements to a cell parameter measurement from a reference embryo/pluripotent cell to determine likelihood of reaching the blastocyst stage and/or usable blastocyst stage for each embryo/pluripotent cell, and then comparing the determination of the likelihood of reaching the blastocyst stage and/or usable blastocyst stage for each embryo/pluripotent cell to determine the likelihood of reaching the blastocyst stage and/or usable blastocyst stage of the embryos or pluripotent cells relative to one another.

In this way, a practitioner assessing, for example, multiple zygotes/embryos, can choose only the best quality embryos, i.e. those with the best likelihood of reaching the blastocyst stage and/or usable blastocyst stage, to transfer so as to maximize the chance of success of a full term pregnancy while minimizing risk. Conversely, the practitioner can minimize the risk of transferring an embryo that is not likely to lead to a successful pregnancy by deselecting embryos determined to be unlikely reach the blastocyst stage or usable blastocyst stage.

Also provided are reagents, devices and kits thereof for practicing one or more of the above-described methods. The subject reagents, devices and kits thereof may vary greatly. Reagents and devices of interest include those mentioned above with respect to the methods of measuring any of the aforementioned cell parameters, where such reagents may include culture plates, culture media, microscopes, imaging software, imaging analysis software, nucleic acid primers, arrays of nucleic acid probes, antibodies, signal producing system reagents, etc., depending on the particular measuring protocol to be performed.

In addition to the above components, the subject kits will further include instructions for practicing the subject methods. These instructions may be present in the subject kits in a variety of forms, one or more of which may be present in the kit. One form in which these instructions may be present is as printed information on a suitable medium or substrate, e.g., a piece or pieces of paper on which the information is printed, in the packaging of the kit, in a package insert, etc. Yet another means would be a computer readable medium, e.g., diskette, CD, etc., on which the information has been recorded. Yet another means that may be present is a website address which may be used via the internet to access the information at a removed site. Any convenient means may be present in the kits.

Some of the methods described above require the ability to observe embryo and stem cell development via time-lapse imaging. This can be achieved using any system capable of time lapse imaging including the Eeva system described in WO 2012/047678, the Embryoscope described in US 2010/041090; US 2012/0309043; US 2013/0102837; US 2011/0183367; US 2011/01656909; US 2011/0111447; WO 2012/163363; WO 2013/004239; WO 2013/029625 and the Primovision system described in US 2012/0140056, or any other time lapse imaging system capable of analyzing and/or measuring the claimed parameters and morphological features of an embryo. Each of these references is incorporated by reference herein in their entirety. This can be achieved using a system comprised of a miniature, multi-channel microscope array that can fit inside a standard incubator. This allows multiple samples to be imaged quickly and simultaneously without having to physically move the dishes. One illustrative prototype, shown in Fig. 22 of US Patent No. 7,963,906, consists of a 3-channel microscope array with darkfield illumination, although other types of illumination could be used. By "three channel," it is meant that there are three independent microscopes imaging three distinct culture dishes simultaneously. A stepper motor is used to adjust the focal position for focusing or acquiring 3D image stacks. White-light LEDs are used for illumination, although we have observed that for human embryos, using red or near-infrared (IR) LEDs can improve the contrast ratio between cell membranes and the inner portions of the cells. This improved contrast ratio can help with both manual and automated image analysis. In addition, moving to the infrared region can reduce phototoxicity to the samples. Images are captured by low-cost, high-resolution webcams, but other types of cameras may be used.

As shown in Fig. 22 of US Patent No. 7,963,906, each microscope of the prototype system described above is used to image a culture dish which may contain anywhere from 1-30 embryos. The microscope collects light from a white light LED connected to a heat sink to help dissipate any heat generated by the LED, which is very small for brief exposure times. The light passes through a conventional dark field patch for stopping direct light, through a condenser lens and onto a specimen labeled "petri dish," which is a culture dish holding the embryos being cultured and studied. The culture dish may have wells that help maintain the order of the embryos and keep them from moving while the dish is being carried to and from the incubator. The wells can be spaced close enough together so that embryos can share the same media drop. The scattered light is then passed through a microscope objective, then through an achromat doublet, and onto a CMOS sensor. The CMOS sensor acts as a digital camera and is connected to a computer for image analysis and tracking as described above.

This design is easily scalable to provide significantly more channels and different illumination techniques, and can be modified to accommodate fluidic devices for feeding the samples. In addition, the design can be integrated with a feedback control system, where culture conditions such as temperature, CO2 (to control pH), and media are optimized in real-time based on feedback and from the imaging data. This system was used to acquire time-lapse videos of human embryo development, which has utility in determining embryo viability for in vitro fertilization (IVF) procedures. Other applications include stem cell therapy, drug screening, and tissue engineering.

In one embodiment of the device, illumination is provided by a Luxeon white light-emitting diode (LED) mounted on an aluminum heat sink and powered by a BuckPuck current regulated driver. Light from the LED is passed through a collimating lens. The collimated light then passes through a custom laser-machined patch stop, as shown in Fig. 22 of US Patent No. 7,963,906, and focused into a hollow cone of light using an aspheric condenser lens. Light that is directly transmitted through the sample is rejected by the objective, while light that is scattered by the sample is collected. In one embodiment, Olympus objectives with 20X magnification are used, although smaller magnifications can be used to increase the field-of-view, or larger magnifications can be used to increase resolution. The collected light is then passed through an achromat doublet lens (i.e. tube lens) to reduce the effects of chromatic and spherical aberration. Alternatively, the collected light from the imaging objective can be passed through another objective, pointed in the opposing direction, that acts as a replacement to the tube lens. In one configuration, the imaging objective can be a 10X objective, while the tube-lens objective can be a 4X objective. The resulting image is captured by a CMOS sensor with 2 megapixel resolution (1600 x 1200 pixels). Different types of sensors and resolutions can also be used.

For example, Fig. 23A of US Patent No. 7,963,906 shows a schematic of the multi-channel microscope array having 3 identical microscopes. All optical components are mounted in lens tubes. In operation of the array system, Petri dishes are loaded on acrylic platforms that are mounted on manual 2-axis tilt stages, which allow adjustment of the image plane relative to the optical axis. These stages are fixed to the base of the microscope and do not move after the initial alignment. The illumination modules, consisting of the LEDs, collimator lenses, patch stops, and condenser lenses, are mounted on manual xyz stages for positioning and focusing the illumination light. The imaging modules, consisting of the objectives, achromat lenses, and CMOS sensors, are also mounted on manual xyz stages for positioning the field-of-view and focusing the objectives. All 3 of the imaging modules are attached to linear slides and supported by a single lever arm, which is actuated using a stepper motor. This allows for computer-controlled focusing and automatic capture of image-stacks. Other methods of automatic focusing as well as actuation can be used.

The microscope array was placed inside a standard incubator, as shown in, for example, Fig. 23B of US Patent No. 7,963,906. The CMOS image sensors are connected via USB connection to a single hub located inside the incubator, which is routed to an external PC along with other communication and power lines. All electrical cables exit the incubator through the center of a rubber stopper sealed with silicone glue.

The above described microscope array, or one similar, can be used to record time-lapse images of early human embryo development and documented growth from zygote through blastocyst stages. In some embodiments, images can be captured every 5 minutes with roughly 1 second of low-light exposure per image. The total amount of light received by the samples can be equivalent to 24 minutes of continuous exposure, similar to the total level experienced in an IVF clinic during handling. The 1 second duration of light exposure per image can in some embodiments be reduced. Prior to working with the human embryos, we performed extensive control experiments with mouse pre-implantation embryos to ensure that both the blastocyst formation rate and gene expression patterns were not affected by the imaging process.

Individual embryos can be followed over time, even though their positions in the photographic field shifted as the embryos underwent a media change, in some cases the media was changed at day 3. The use of sequential media is needed to meet the stage-specific requirements of the developing embryos. During media change, the embryos were removed from the imaging station for a few minutes and transferred to new petri dishes. In order to keep track of each embryo's identity during media change, the transfer of samples from one dish to the other was videotaped to verify that embryos were not mixed up. This process was also used during the collection of samples for gene expression analysis. The issue of tracking embryo identity can be mitigated by using wells to help arrange the embryos in a particular order.

Petri dish with micro-wells

When transferring the petri dishes between different stations, the embryos can sometimes move around, thereby making it difficult to keep track of embryo identity. This poses a challenge when time-lapse imaging is performed on one station, and the embryos are subsequently moved to a second station for embryo selection and transfer. One method is to culture embryos in individual petri dishes. However, this requires each embryo to have its own media drop. In a typical IVF procedure, it is usually desirable to culture all of a patient's embryos on the same petri dish and in the same media drop. To address this problem, we have designed a custom petri dish with micro-wells. This keeps the embryos from moving around and maintains their arrangement on the petri dish when transferred to and from the incubator or imaging stations. In addition, the wells are small enough and spaced closely together such that they can share the same media drop and all be viewed simultaneously by the same microscope. The bottom surface of each micro-well has an optical quality finish. For example, Fig. 27A in US Patent No. 7,963,906 shows a drawing with dimensions for one exemplary embodiment. In this version, there are 25 micro-wells spaced closely together within a 1.7 x 1.7 mm field-of-view. Fig. 27B of US Patent No. 7,963,906 shows a 3D-view of the micro-wells, which are recessed approximately 100 microns into the dish surface. Fiducial markers, including letters, numbers, and other markings, are included on the dish to help with identification. All references cited herein are incorporated by reference in their entireties.

EXAMPLES

The following examples are put forth so as to provide those of ordinary skill in the art with a disclosure and description of how to make and use the present invention, and are not intended to limit the scope of what the inventors regard as their invention nor are they intended to represent that the experiments below are all or the only experiments performed. Efforts have been made to ensure accuracy with respect to numbers used (e.g. amounts, temperature, etc.) but some experimental errors and deviations should be accounted for. Unless indicated otherwise, parts are parts by weight, molecular weight is weight average molecular weight, temperature is in degrees Centigrade, and pressure is at or near atmospheric.

EXAMPLE 1

This example describes the development of a blastocyst prediction model and its utility in an IVF clinic.

METHODS

To develop the blastocyst prediction model, a clinical study was performed to collect data from 3 sites, 54 subjects and 292 embryos. The embryos were cultured using standard procedures in an IVF lab and imaged at 5 minute intervals inside the incubator. By retrospectively analyzing the image data, it was shown that quantification of the timing of early cell division up to approximately 48 hours after fertilization could predict whether an embryo would become a blastocyst on day 5 with a high degree of specificity. During this analysis, it was found that the time between 1st and 2nd mitosis (p2) and the time between 2nd and 3rd mitosis (p3) significantly contributed to the predictive power of the prediction model. Therefore, the blastocyst prediction model was based on the time between 1st and 2nd mitosis (p2) and the time between 2nd and 3rd mitosis (p3).

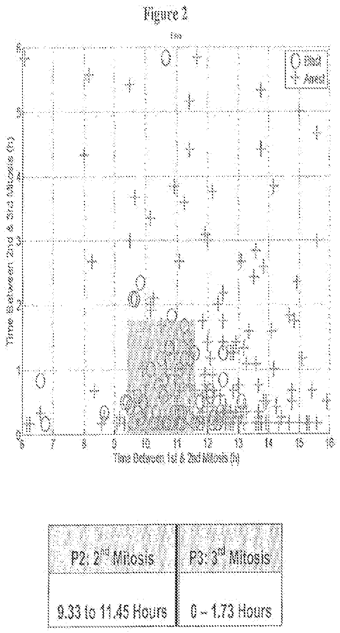

Figure 2 shows a plot of all embryos in the development study, with the range of P2 times plotted along the horizontal axis, and the P3 times plotted on the vertical axis. The accompanying table show time frames for P2 and P3 that were found to be predictive of blastocyst formation.

### TABLE 1. P2 AND P3 PREDICTIVE RANGES.

| P2: 2nd Mitosis | P3: 3rd Mitosis |
|---|---|
| 9.33 to 11.45 Hours | 0 - 1.73 Hours |

In clinical use of the blastocyst prediction model, the measurements of the P2 and P3 events are compared to the validated blastocyst predictive time windows. The measurements of the parameters can be performed manually by reviewing the images, semiautomatically with software assistance or annotation tools, or completely automated using image analysis software. If both events are within the predictive windows, the model predicts the embryo has a High Probability of reaching the blastocyst stage. If one or both of the events fall outside of the predictive windows, the model predicts that the embryo has a Low Probability of reaching the blastocyst stage.

INTERPRETATION OF DATA

In the clinical embryology laboratory setting, the standard embryo selection process for a clinical embryologist (CE) begins with evaluation of the cohort of embryos with an assisted reproductive microscope. The morphology information is captured on a laboratory worksheet, and the embryos are returned to the incubator. Next, the worksheet information is used to categorize or "rank" embryos. CEs review the data from the cohort of embryos and generally follow one of two ranking strategies.

Ranking Strategy 1: If the majority of embryos are of *good morphology,* the CE will (1) "de-select" the poorest quality embryos from further consideration, (2) select the top embryo(s) for transfer, and (3) determine which of the remaining embryos will be cryopreserved.

Ranking Strategy 2: If the majority of embryos are of *poor morphology,* the CE will (1) select the top embryos(s) for transfer, (2) identify the embryos to not transfer, and (3) determine which of the embryos will be cryopreserved.

The critical challenge for this selection process occurs when a patient has more good morphology embryos than the number of embryos planned for transfer. It is known that when prospectively evaluating embryos in the clinical setting, almost 50% of embryos with good Day 3 morphology do not progress to become blastocysts by Day 5. Alternately, looking retrospectively, 80% of embryos that become blastocysts exhibit good Day 3 morphology. As a result, embryo selection using traditional morphology is characterized by a high false positive prediction rate. In other words, traditional morphology has a high sensitivity for identifying good morphology embryos on Day 3, but very low specificity for selecting among the good morphology embryos those that will progress to the blastocyst stage and are good candidates for transfer.

<u>EXAMPLE 2</u>

PURPOSE

This example describes the process used to develop statistical classification models for predicting blastocyst formation based on the blastocyst prediction timing parameters.

Model Development

The clinical study dataset was collected to help build and evaluate different types of statistical classification models for predicting blastocyst formation. The input parameters to these classifiers were the 3 predictive parameters (based on the paper Wong CC, Loewke KE, Bossert NL, Behr B, De Jonge CJ, Baer TM, Reijo Pera RA. Non-Invasive Imaging of Human Embryos Before Embryonic Genome Activation Predicts Development to the Blastocyst Stage. Nat Biotechnol. 2010 Oct;28(10):1115-21.): duration of first cytokinesis (P1), time between 1st and 2nd mitosis (P2), and time between 2nd and 3rd mitosis (P3).

The models were trained on an extensive clinical study dataset The dataset consisted of 292 embryos across 45 patients. The average age of the egg is 33.6 ± 4.8. There are 25 subjects with 143 embryos that used the insemination method of ICSI and 18 subjects with 138 embryos that used the insemination method IVF. There were 2 subjects with 11 embryos that used both ICSI and IVF.

**Table 2: Represents the Day 3 Quality of the embryos:**

|  | Day 3 Quality | Overall |
|---|---|---|
| **Total Number of Embryos** |  | 292 |
| **Cells** | Mean Cells ± SD | 6.7 ± 1.9 |
| **Fragmentation** | No Fragmentation | 58/292 (20%) |
|  | 1-10% | 130/292 (45%) |
|  | 11-25 % | 81/292 (28%) |
|  | >25% | 23/292 (8%) |
| **Symmetry** | Perfect | 169/292 (58%) |
|  | Moderate Asymmetric | 101/292 (34%) |
|  | Severely Asymmetric | 22/292 (7%) |
| **Grade** | Good | 156/292 (54%) |
|  | Fair | 97/292 (33%) |
|  | Poor | 39/292 (13%) |

Blastocyst Outcome

The definition used for "blastocyst" in this study was embryos that formed blastocysts on day 5 (i.e., usable blastocysts) and were subsequently either transferred or frozen. Embryos that did not meet the definition of blastocyst were counted as Arrested. For example, an embryo that did not form a blastocyst on day 5, or formed a blastocyst on day 5 but was subsequently not transferred, would be called Arrested for this example. This definition was used to focus on building predictive models for good-quality or 'usable' blastocysts. Based on these definitions, the prevalence of usable blastocyst formation in the development dataset is 23%.

Parameter Measurements

A panel of 3 expert clinical embryologists was assembled. Each embryologist independently reviewed the data from all embryos in the Development Dataset that were cultured to Day 5. The embryologists were blinded to the study site, any identifying subject data, total number of embryos per subject, and the predictions from the blastocyst prediction model or the other members of the panel. The order of the embryos presented to the panel members was randomized from the entire pool of evaluable embryos from all subjects. Each reviewer received a separate randomization worklist using the same embryos.

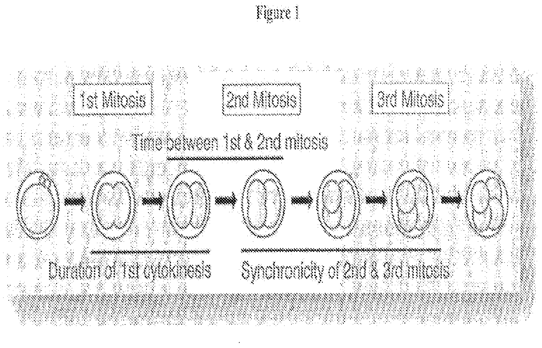

Using an image movie viewer, each panel member reviewed all embryos in the Study Group. They evaluated the embryos one at a time and attempted to identify the image frame, and the specific start and stop time for each of the 3 development events (Figure 1):

    1. Start Time P1
    2. Stop Time P1 / Start Time P2
    3. Stop Time P2 / Start Time P3
    4. Stop Time P3

P1 is defined as the duration of first cytokinesis.

P2 is defined as the time interval between the first and second mitosis (also refered to as the time of division from 2-cells to 3-cells or the time interval between cytokinesis 1 and cytokinesis 2).

P3 is defined as the time interval between the second and third mitosis (also refered to as the time of division from 3-cells to 4-cells or the time interval between cytokinesis 2 and cytokinesis 3).

If the panel member determined that the embryo did not achieve a development event (i.e. embryo stalls at some development point or arrests) then that development time point was recorded as a "no-event."

If an embryo was visible but the image quality was insufficient for the panel member to make a judgment of the embryo status (i.e. out of focus, insufficient lighting, etc.) then that was indicated as "poor image quality."

For each embryo, the results from the panel were exported to a CSV file. The CSV file contained the start/stop times and the elapsed time, or a no-event for each of the events individually from the panel embryologists.

MODELS

Several types of models were explored, such as classification trees, random forests, linear and quadratic discriminant analysis, and Naive Bayes. The models described in this example are exemplary models.

All four of the candidate models were based on two timing parameters that contributed significantly to the predictive power of the models: the time between 1st and 2nd mitosis (P2), and the time between 2nd and 3rd mitosis (P3).

Classification Tree Model: There are 2 variations of the Classification Tree model

Model 1: Classification Tree Model with empirically-learned Priors. The minparent (i.e. the number K such that impure nodes must have K or more observations to be split) was set to 50.

Model 2: Classification Tree Model with equal (50/50) Priors. The minparent (i.e. the number K such that impure nodes must have K or more observations to be split) was set to 75.

Naive Bayes Model: There are 2 variations of the Naive Bayes model

A Naive Bayes classifier assigns a new observation to the most probable class, assuming the features are conditionally independent given the class value.

Model 3: Naive Bayes with Gaussian model and probability cutoff of .4041.

Model 4: Naive Bayes with Gaussian model and probability cutoff of .2944. Model selection for validation

Model 2 was chosen for the blastocyst prediction model for this example. After evaluating the four models, we make the following observations:

1. The classification tree models may be preferred due to their simplicity and similarity to the model used in Wong et al.
2. The cross validation errors for both of the classification tree models are very similar and therefore either model can be supported.
3. The sensitivity and specify of 68%, and 83%, respectively, of Model 2 allow for a high specificity model.
4. The timing windows predicted by the Model 2 are highly relevant based on the biology of embryo development and preliminary pregnancy data (data not shown).

Model 1: Parameters used in this example

Classification Tree
Minparent = 50
Prior = empirical
Performance on Training Data:

    Sensitivity = 57%
    Specificity = 88%
    PPV = 59%
    NPV = 87%

Misclassification rate (on Training Data): 19%
10-fold Cross-validation misclassification rate: 25%

The cross-validation procedure was performed in Matlab. The method partitions the sample into 10 subsamples, chosen randomly but with roughly equal size. The subsamples also have roughly the same class proportions. For each subsample, the method fits a tree to the remaining data and uses it to predict the outcome in the subsample. It pools the information from all subsamples to compute the misclassification rate for the whole sample.

Model 2: Parameters used in this example

Classification Tree
Minparent = 75
Prior = equal (50/50)
Performance on Training Data:

Sensitivity = 68%
Specificity = 83%
PPV = 55%
NPV = 89%

Misclassification rate (on Training Data): 25%
10-fold Cross-validation misclassification rate: 30%

The cross-validation procedure was performed in Matlab. The method partitions the sample into 10 subsamples, chosen randomly but with roughly equal size. The subsamples also have roughly the same class proportions. For each subsample, the method fits a tree to the remaining data and uses it to predict the outcome in the subsample. It pools the information from all subsamples to compute the misclassification rate for the whole sample.

Model 3: Naive Bayes Parameters used in this example

Class prior probability P(blast)=0.3024

E(P2|blast)=10.8454
E(P3|blast)=0.5381

$$\sigma^2_{P2|blast} = 0.859$$

$$\sigma^2_{P3|blast} = 0.2191$$

E(P2|arrest)=11.8749
E(P3|arrest)=0.6716

$$\sigma^2_{P2|arrest} = 1.8873$$

$$\sigma^2_{P3|arrest} = 0.3807$$

Probability cutoff = 0.4041
AUC on training data: 0.793
Performance on Training Data for cutoff of 0.4041:

Sensitivity = 62%
Specificity = 83%
PPV = 53%
NPV = 88%

Model 4: Naive Bayes Parameters used in this example

class prior probability P(blast)=0.3024

E(P2|blast)=10.8454
E(P3|blast)=0.5381

$$\sigma^2_{P2|blast} = 0.859$$

$$\sigma^2_{P3|blast} = 0.2191$$

E(P2|arrest)=11.8749
E(P3|arrest)=0.6716

$$\sigma^2_{P2|arrest} = 1.8873$$

$$\sigma^2_{P3|arrest} = 0.3807$$

Probability cutoff = 0.2944
AUC on training data: 0.793
Performance on Training Data for cutoff of 0.2944:

Sensitivity = 68%
Specificity = 77%
PPV = 47%
NPV = 89%

EXAMPLE 3

Development and validation of a new test for predicting embryo viability based on time-lapse imaging and automated cell tracking.

ABSTRACT

The objective of this study was to develop and prospectively validate a new, real-time early embryo viability assessment platform for improving embryo selection in in vitro fertilization (IVF) laboratories.
The specificity, positive predictive value and overall accuracy of identifying Usable Blastocysts (blastocysts deemed suitable for transfer or freezing) at the cleavage stage are significantly improved when using the new test compared to traditional Day 3 morphology.
New embryo selection methods are expected to improve IVF success rates and reduce the need for multiple embryo transfer, yet step-by-step approaches to validate new technology for clinical usefulness are lacking. In this study, scientifically-based time-lapse image markers are integrated with cell tracking capabilities to create the first method for quantitatively measuring embryos and generating blastocyst predictions in real-time, and the method is independently validated for diagnostic accuracy and clinical utility.
This was a prospective, multi-center, single arm, nonrandomized, cohort study conducted between June 2011 and February 2012. The study was designed to collect imaging data of embryos followed to the cleavage (Day 3) or blastocyst (Day 5) stage, in order to characterize the safety and efficacy of the *Eeva™* (Early Embryo Viability Assessment) System for predicting which embryos would develop to Usable Blastocysts. A total of 160 patients consented to have their embryos imaged using Eeva. Experienced embryologists were blinded to the embryo outcome, and independently reviewed videos for specific cell division time intervals from the 1- to 4-cell stage, P1 (duration of first cytokinesis), P2 (time between cytokinesis 1 and 2) and P3 (time between cytokinesis 2 and 3). A classification tree was built to predict Usable Blastocysts based on these intervals, and a cell tracking software system was developed to automatically measure cell division timings and generate real-time predictions of embryo development. The prediction and cell tracking software capabilities were validated on an independent set of 1,029 embryos and assessed for performance.
Since the outcome measure of this study was blastocyst formation, study inclusion criteria were: women at least 18 years of age undergoing fresh IVF treatment using their own eggs or donor eggs, basal antral follicle count (AFC) of at least 8 as measured by ultrasound prior to stimulation, basal follicle stimulating hormone (FSH) < 10 IU, and at least 8 normally fertilized oocytes (2PN). The study was conducted at five IVF clinical sites in the U.S.

Eeva was statistically determined to predict a high probability of Usable Blastocyst development when both P2 and P3 are within specific cell division timing ranges (9.33≤P2≤11.45 hours and 0≤P3≤1.73 hours). Prospectively using Eeva on an independent Validation Dataset, the specificity and positive predictive value (PPV) for blastocyst prediction was significantly improved over the average prediction made by experienced embryologists using Day 3 morphology (specificity 84.7% vs. 57.0%, p<0.0001; PPV 54.7% vs. 33.7%, p<0.0001). The sensitivity for blastocyst prediction was 38.0% (95% CI of 32.7% to 43.5%), and the NPV was 73.7% (95% CI of 70.4% to 76.8%). The cell tracking software was determined to have an overall agreement with manual measurements and predictions of 91.0% (95%CI of 86.0% to 94.3%).

The study outcome of blastocyst formation required testing on a patient population whose embryos were cultured to blastocysts; therefore, the validation of Eeva's performance may be representative of a better prognosis patient group. The characteristics of the embryos from patients with less than 8 antral follicles and fewer than 8 2PNs will be addressed in future studies.

We have developed and validated an early embryo viability assessment platform which identifies Usable Blastocysts by tracking quantitative measurements of key cell division timings to the cleavage stage. Eeva predictions are non-invasive, specific and easily integrated into the workflow of Day 3 or Day 5 transfer procedures. These results represent a solid step in the rigorous study, evaluation and validation of a new, real-time embryo selection platform for use in the IVF clinic.

New embryo selection methods are expected to improve in vitro fertilization (IVF) pregnancy rates and result in broader adoption of single embryo transfer (van Montfoort *et al*., 2005). Embryo assessment is currently based on the highly subjective and variable morphological evaluation of only a few static snapshots of the embryo during its development. However, it is well recognized that traditional morphology has limited accuracy and specificity for identifying the best embryos. Embryologists are consequently faced with great difficulty in discriminating among good morphology embryos those with highest developmental competence.

Here we present a clinical study for the development and validation of a new Early Embryo Viability Assessment (Eeva) platform based on non-invasive time-lapse imaging and validated cell division timings. The study extends upon seminal scientific findings that demonstrated that strict cell cycle division timings can both predict embryo development and reflect the underlying health of preimplantation human embryos (Wong *et al*., 2010). In the study, time-lapse imaging was used to investigate an array of dynamic, morphologic, and quantitative measures of preimplantation human embryo development. A small set of early cell division parameters that accurately predicted viable blastocyst formation were identified, and the key parameters were also probed for significance at the gene expression level. The objectives of the current, prospective clinical study were to (1) validate the predictive power of those cell division timings in clinical settings, using Usable Blastocysts (blastocysts deemed suitable for transfer or freezing) as the outcome, (2) develop software to reliably track cell division timings to enable practical clinical utility, (3) demonstrate the feasibility of successfully tracking the overwhelming majority of embryos imaged, and (4) characterize the diagnostic accuracy of the integrated system on an independent set of embryos, important steps towards bringing Eeva to the IVF clinic.

## MATERIALS & METHODS

This was a prospective, blinded, single-arm, nonrandomized, clinical study conducted at five IVF clinical sites in the United States between June 2011 and February 2012, comprised of two sequential components, a Developmental phase and a Validation phase. The study was designed to collect imaging data of embryos followed to the cleavage (Day 3) or blastocyst (Day 5) stage, in order to characterize the safety and efficacy of the Eeva System. The clinical investigation plan was approved by an Institutional Review Board (IRB), and registered at ClinicalTrials.gov (study number NCT01369446). Written informed consent was obtained from all study participants. Patients who met eligibility criteria for the study's Development phase were: women at least 18 years of age undergoing fresh IVF treatment using their own eggs or donor eggs, basal antral follicle count (AFC) of at least 8 as measured by ultrasound prior to stimulation, and basal follicle stimulating hormone (FSH) < 10 IU. For the study's Validation phase, patients who met eligibility criteria for the study's Validation phase were: women at least 18 years of age undergoing fresh IVF treatment using their own eggs or donor eggs, basal antral follicle count (AFC) of at least 12 as measured by ultrasound prior to stimulation, basal follicle stimulating hormone (FSH) < 10 IU, and at least 8 normally fertilized oocytes (2PN). The study inclusion criteria for the Validation phase were designed to capture the patient population who planned to culture their embryos to blastocysts, while the inclusion criteria for the Development phase were less limiting and included women with day 3 embryo transfer. The criteria for exclusion of patients in both phases were those who: used a gestational carrier, used surgically removed sperm, used re-inseminated oocytes, planned preimplantation genetic diagnosis or preimplantation genetic screening, were concurrently participating in another clinical study, had previously enrolled in this clinical study, or had history of cancer treatment.

## Ovarian stimulation, fertilization and embryo culture

Each clinical site followed their standard procedures for ovarian stimulation, oocyte pickup, fertilization and embryo culture. Patients underwent ovarian stimulation according to guidelines of each clinic, where protocols included agonist luteal phase, agonist micro-dose flare, and antagonist suppression. On the day of oocyte retrieval (Day 0), oocytes were fertilized using the clinical site's discretion of conventional IVF or intracytoplasmic sperm injection (ICSI). Immediately following the fertilization check, successfully fertilized oocytes (2PNs) were transferred to a multiwell Eeva dish for culture and monitoring in a standard incubator at 37°C. The Eeva dish is a standard 35-mm diameter petri dish made of conventional tissue culture plastic, with an inner ring containing a precision-molded array of 25 wells (well size 250 $\mu$m length x 250 $\mu$m width x 100 $\mu$m depth). The microwell format holds individual embryos separately but in close proximity to each other under a shared media droplet (40 $\mu$l overlaid with mineral oil), while fiducial labels provide a visual reference of each embryo's specific location in the dish array. Individual well tracking is performed under a single optical field of view, which reduces the need for motorized parts, used often in imaging systems to individually address and monitor each embryo (Vajta *et al*., 2000; Sugimura *et al*., 2010; Cruz *et al*., 2011; Meseguer *et al*., 2011; Hashimoto *et al*., 2012). At the same time, the shared media permits group culture, which may improve blastocyst formation rates by promoting positive paracrine signaling between embryos (Rijnders *et al*., 1999; Blake *et al*., 2007). Throughout embryo culture, each clinical site was allowed to use their own laboratory protocols, including their standard culture media and incubation environment (e.g., $CO_2$ in air or low $O_2$).

## Embryo imaging

Images of developing embryos were captured with the *Eeva™* (Early Embryo Viability Assessment) System, an integrated time-lapse imaging system encompassing: (1) the Eeva dish for culturing a cohort of embryos, (2) a digital, inverted time-lapse microscope with darkfield illumination, auto-focus and 5 megapixel camera, and (3) image acquisition software to capture images during embryo development and to save the images to file. The Eeva microscope captures a single, high resolution image of all the micro-wells in the petri dish once every 5 minutes. During the analysis process, the image acquisition software segments the images into a series of sub-images. The analysis is performed separately for each embryo, and the computation is parallelized so all embryos across all microscopes can be processed in real-time.

Eeva was designed to record embryo development with minimal light exposure to embryos from a light-emitting diode at 625 nm wavelength. Using an optical power meter, it was determined that the power of the illuminating LED light of the Eeva Microscope is approximately 0.6 milli-watts/cm$^2$. By comparison, the power of a typical IVF inverted microscope (measured on the Olympus IX-71 and CK40 Hoffman Modulation Contrast systems) can be up to 10 milli-watts/cm$^2$. Eeva captures a relatively high image frequency (one image every 5 minutes), at a relatively low light intensity and exposure time (0.6 seconds for each image). Thus, Eeva produces only 0.36 milli-joules/cm$^2$ of energy per image, or a total energy exposure of only 0.32 joules/cm$^2$ over 3 days of imaging. Altogether, the total light energy experienced by embryos during 3 days of Eeva imaging approximates 21 seconds exposure from a traditional IVF bright field microscope. The duration of Eeva imaging from post-fertilization check to Day 3 produces approximately 865 image frames per embryo.

During the imaging process, no media change or observation was allowed. Imaging was continued through Day 3 and stopped at the time of routine Day 3 embryo grading.

## Morphological grading

Following the completion of Eeva imaging on Day 3, the remainder of the IVF process was continued per conventional procedures at each site. Day 3 embryo grading was performed according to the clinic's standard protocols. The embryologist used traditional morphology criteria to decide which embryos were selected for transfer, extended culture, freezing, or discard. If the case was designated for blastocyst culture, the embryos were moved from the Eeva dish to a regular culture dish, and blastocyst culture was carried out based on the clinic's standard protocols for Day 5 or Day 6 morphological grading and blastocyst transfer.

Recording formats for embryo morphological grading vary among clinics; therefore, embryo morphological grading data, for both the cleavage stage and blastocyst stage, were collected using the Society of Assisted Reproductive Technologies (SART) standard (Racowsky *et al*., 2010; Vernon *et al*., 2011). Embryo fate, recorded as "transferred", "frozen" or "discarded", was collected at each clinical site according to each site's own established protocol.

## Data management and manual measurements of cell division timings

Raw image data collected from the sites was segregated into two distinct datasets for each phase of the study: a Development Dataset (n=736 embryos from 63 patients) and a separate, sequestered Validation Dataset (n=1,029 embryos from 75 patients). No patient was represented in both datasets; rather, all embryo images from an individual patient were only added to either dataset. An image database tool was employed to (1) compile images into a time-lapse video with well identification labels and timestamps, (2) enable video playback, and (3) allow manual annotation of the start/stop times of notable developmental events. A panel of three embryologists independently reviewed embryo videos following a blinded, randomized protocol. For each embryo, each panelist recorded the start/stop times of specific cell division time intervals from the 1-to-4-cell stage which were previously reported to predict successful development to the blastocyst stage: P1 (duration of first cytokinesis), P2 (time between cytokinesis 1 and 2) and P3 (time between cytokinesis 2 and 3) (Wong *et al.*, 2010). Each embryologist was blinded to any patient data, including the total number of embryos per patient, prediction results from Eeva or the measurements of any other embryologist.

**Prediction and cell tracking software**

Development of the Eeva prediction and embryo cell tracking software was completed using a subset of n=292 embryo videos from 43 patients in the Development Dataset. First, a classification tree model was built to assess the predictive capability of P1, P2, and P3 measurements for a specific outcome of embryo development: Usable Blastocyst formation. Usable Blastocysts were defined as embryos that were morphologically graded to be blastocysts on Day 5 or Day 6, and were of sufficient quality that they were selected for transfer or freezing by embryologists from the clinical sites. Embryos that did not meet the definition of Usable Blastocyst were counted as "Arrested" as they were discarded by the embryologists from the clinical sites.

To automate the measurement of the parameters, software for cell tracking was developed using a data driven probabilistic framework and computational geometry to track cell division from the 1-cell to 4-cell stage. The primary features tracked by the algorithm are cell membranes, which exhibit high image contrast through the use of darkfield illumination. The software generates an embryo model that includes an estimate of the number of blastomeres, as well as blastomere size, location, and shape, as a function of time. Parameter measurements from the embryo models are fed through the classification tree that predicts Usable Blastocyst formation.

The prediction model and cell tracking software were tested on an independent Validation Dataset of n=1,029 embryos from 75 patients to evaluate accuracy and robustness.

**Statistical analyses**

All data and statistical analyses were carried out using SAS Software version 9.2 and Matlab version R2010a. The statistical classification tree model was built to determine how well "Usable Blastocysts" and "Arrested" embryo parameter timings split at nodes defined by P1, P2 and P3 cell division timing parameters. The model was trained on 292 embryos with manual measurements of the parameters and known blastocyst outcomes. To test non-inferiority between manual and software measurements, methods of Blackwelder were utilized with power $(1-\beta)\% = 0.8$ and significance $\alpha\% = 0.05$. Overall percent agreement between the two methods was also determined using a method agreement analysis. Diagnostic measures (e.g., specificity, sensitivity, PPV, NPV) and associated 95% confidence intervals were calculated to assess performance of predicting Usable Blastocyst outcome. To compare the performance of morphology-based predictions to Eeva predictions, a proportions test was performed. A value of $p < 0.05$ was considered statistically significant.

**RESULTS**

**Clinical characteristics**

A total of 160 patients at 5 IVF clinical sites met eligibility criteria and consented to have their embryos imaged using the Eeva system. Altogether, 2,682 oocytes were retrieved and fertilized by IVF or ICSI. Following fertilization, 1,765 were confirmed 2PNs and transferred to Eeva dishes for imaging from the post-fertilization to Day 3 stage. At the completion of Eeva imaging on Day 3, traditional Day 3 morphology grading was collected for 1,727 embryos. According to the standard protocol of each clinical site, some embryos were selected for transfer while other embryos were cultured an additional two days. Day 5 morphology was collected for 1,494 embryos and used to calculate the overall blastocyst formation (838/1,494 = 56%) and Usable Blastocyst formation (443/1,494 = 30%), defined as the formation of blastocysts that were of sufficient quality such that they were selected for transfer or freezing **(Figure 7).**

Embryos that were usable in the prediction and cell tracking software Development and Validation phases were embryos that were cultured to the blastocyst stage. Of the 160 enrolled patients, 22 were excluded from Development and Validation: the first 2 or 3 cases from each site were allocated to training and ensuring proper use of the Eeva system (total 12 cases), and an additional 10 patients were Day 3 transfer cases with incomplete blastocyst outcome data. The clinical characteristics of the 138 remaining patients and embryos in both datasets are summarized in **Table 3.**

**Table 3:** Clinical characteristics of Development and Validation Datasets. *"Other" includes 11 reasons: 3 of age-related sub-fertility; 2 due to oligoovulation; 2 due to the subject being a single female; 1 due to amenorrhea; 1 due to menopause; 1 due to recurrent pregnancy loss; and 1 due to tubal adhesions.

**TABLE 3: CLINICAL CHARACTERISTICS OF DEVELOPMENT AND VALIDATION DATASETS.**

| Clinical Characteristics | | Development Dataset | Validation Dataset |
|---|---|---|---|
| **Total Number of Patients** | | 63 | 75 |
| **Total Number of Eggs** | | 1046 | 1636 |
| **Total Number of 2PNs** | | 736 | 1029 |
| **Patient Demographics** (mean ± SD) | Egg Age (years) | 34.2 ± 4.5 | 32.5 ± 65.4 |
| | Recipient Age (years) | 35.6 ± 4.4 | 35.6 ± 5.6 |
| | Height (inches) | 66.0 ± 2.9 | 65.4 ± 2.9 |
| | Weight (pounds) | 145.1 ± 29.7 | 148.5 ± 32.1 |
| **Cycle Type** | Patient Using Own Eggs | 58/63 (92.1 %) | 62/75 (82.7 %) |
| | Oocyte Donor | 5/63 (7.9%) | 13/75 (17.3 %) |
| **Reason for ART** | Male Infertility | 20/63 (31.8 %) | 15/75 (20.0 %) |
| | History of Endometriosis | 3/63 (4.8 %) | 1/75 (1.3 %) |
| | Ovulation Disorders | 4/63 (6.4 %) | 9/75 (12.0 %) |
| | Diminished Ovarian Reserve | 3/63 (4.8 %) | 10/75 (13.3 %) |
| | Tubal Ligation | 1/63 (1.6 %) | 0/75 (0.0 %) |
| | Tubal Hydrosalpinx | 1/63 (1.6 %) | 0/75 (0.0 %) |
| | Other Tubal Disease | 1/63 (1.6%) | 2/75 (2.7 %) |
| | Uterine | 0/63 (0.0 %) | 1/75 (1.3 %) |
| | Unexplained | 11/63 (17.5 %) | 17/75 (22.7 %) |
| | Multiple Reasons | 11/63 (17.5 %) | 16/75 (21.3 %) |
| | Other* | 8/63 (12.7 %) | 4/75 (5.3 %) |
| **Stimulation Protocol** | Agonist Luteal Phase | 15/63 (23.8 %) | 6/75 (8.0 %) |
| | Agonist Micro-Dose Flare | 2/63 (3.2 %) | 4/75 (5.3 %) |
| | Antagonist Suppression | 29/63 (46.0 %) | 49/75 (65.3 %) |
| | Other | 17/63 (27.0 %) | 16/75 (21.3 %) |
| **Stimulation & Retrieval Counts** (mean ± SD) | AFC Count | 16.9 ± 7.0 | 21.8 ± 8.6 |
| | Number of Follicles | 16.7 ± 7.8 | 21.2 ± 7.5 |
| | Number of Eggs | 16.6 ± 7.3 | 21.8 ± 7.9 |
| **Method of Insemination** | ICSI | 39/63 (61.9 %) | 52/75 (69.3 %) |
| | IVF | 21/63 (33.3 %) | 21/75 (28.0 %) |
| | Both | 3/63 (4.8 %) | 2/75 (2.7%) |
| **Fertilization Count** (mean ± SD) | Number of 2PNs | 9.6 ± 4.7 | 13.7 ± 4.9 |

**Development of Eeva prediction and cell tracking software**

To develop the Eeva system for early embryo viability assessment, 292 embryos from 43 patients with image data, measurement data, and blastocyst outcome data were analyzed and used to build: (1) a statistical classification tree model for predicting Usable Blastocyst outcome, and (2) cell tracking software for measuring predictive cell division timings and generating automated Usable Blastocyst predictions.

The classification tree model provided a simple deterministic path for categorizing embryos as "Usable Blastocysts" or "Arrested" based on optimal ranges of cell division timing parameters. In addition to PI, P2 and P3 cell division timings, other factors were evaluated including egg age, cell number, and method of insemination; however, these were not found to be major predictors of developmental outcome. Further, upon testing methods which included these factors, it was found that P2 and P3 values statistically dominated the prediction. Therefore, the current Eeva prediction and cell tracking software was based on the strongest two of the three previously published timing parameters: the time between 1st and 2nd cytokinesis (P2), and the time between 2nd and 3rd cytokinesis (P3). The Eeva prediction and cell tracking software reported a high probability of Usable Blastocyst formation when both P2 and P3 are within specific cell division timing ranges ($9.33 \leq P2 \leq 11.45$ hours and $0 \leq P3 \leq 1.73$ hours), and a low probability when either P2 or P3 are outside the specific cell division timing ranges (see **Figure 8**).

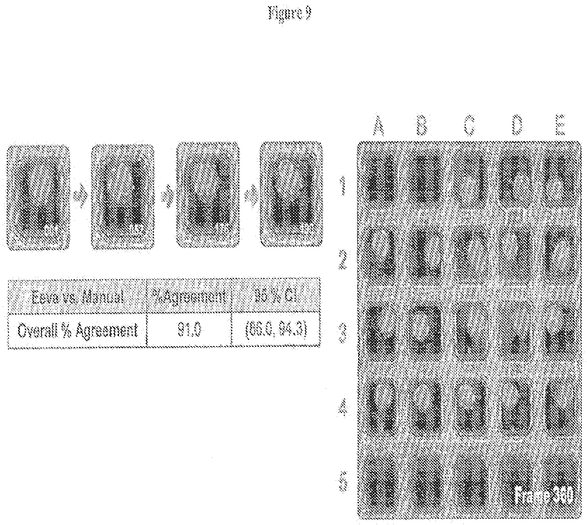

The cell tracking software was implemented in C++ running in real-time on a standard PC. To visualize tracking results, colored rings were overlaid on the original image of the embryo at each stage of cell division, for each frame of the time-lapse sequence (**Figure 9**). The time between cytokinesis 1 and 2 (P2) and the time between cytokinesis 2 and 3 (P3) were calculated by the software and fed through the classification tree model to predict Usable Blastocyst formation by comparing the calculated measurements to reference windows. The software reported a prediction of Usable Blastocyst formation as "high" (for in-window, or high probability) or "low" (for out-window, or low probability) for each embryo.

**Validation of Eeva prediction and cell tracking software**

A prospective, double blind method comparison study was designed to validate the Eeva prediction model and cell tracking accuracy. Validation was completed on an independent set of n= 1,029 embryos from 75 patients, which was segregated from the data used for model development. A method comparison analysis was used to compare the values of the timing parameters and blastocyst predictions of Eeva, compared to an expert embryologist panel. As in the Development phase, three embryologists independently took manual measurements of parameters for embryos in the Validation Dataset. Eeva-generated parameter values and predictions were compared to manual parameter measurements and predictions provided by the three embryologists. Eeva was able to generate measurements and predictions for an overwhelming majority (941/998 = 94.2%) of embryos, and the small fraction that were not suitable for cell tracking were cases which exhibited extremely complex behaviors (e.g., highly abnormal cell divisions and/or high % fragmentation) with primarily Arrested outcomes (45/57=78.9%). Agreement between the embryologist panel and Eeva was assessed and defined as both Eeva and manual methods having "high" (in window) or "low" (outside window) Usable Blastocyst predictions. The overall agreement between the Eeva software and manual measurements in performing Usable Blastocyst predictions was 91.0% (95% CI of 86.0% to 94.3%) (**Figure 9**).

**Outcomes and Eeva predictions for patients and embryo cohorts**

An analysis of the number of "Usable Blastocysts" and "Arrested" embryos for each patient's cohort of embryos was performed and plotted by their P2 measurements and P3 classifications for the Development Dataset (**Figure 10**) and Validation Dataset (**Figure 11**). For this analysis, only the outcomes for all patients who had a complete imaging dataset and all embryos cultured to Day 5 or 6 for blastocyst transfer were evaluated (28 patients for the Development Dataset, 74 patients for the Validation Dataset).

Of the 28 patients shown in the Development Dataset (**Figure 10**), 4 patients had no blastocysts and 24 had at least one blastocyst in their embryo cohort. The prevalence of Usable Blastocysts was 25.2% (=67/266). Most Usable Blastocyst measurements (41/75=54.7%) fell well within the "in-window" range of P2 and P3 cell division timings defined by the classification and regression tree prediction model (depicted in yellow). There was a 17.1% Eeva false positive rate, based on the 34/199 arrested embryos that were within both P2 and P3 ranges. In the Validation Dataset (**Figure 11**), there were 74 patients who had complete Usable Blastocyst outcome and Eeva prediction information for evaluation. In this group, 4 patients had no blastocysts and 70 patients had at least one blastocyst in their cohort of embryos. The total prevalence of Usable Blastocysts was 32.1% (n=320/998 embryos). The total number of Usable Blastocyst that fell well within the "in-window" range of P2 and P3 measurements was 119/308 =38.6%. The Eeva false positive rate was 15.3%, based on the 97/633 arrested embryos that were within both P2 and P3 predictive ranges.

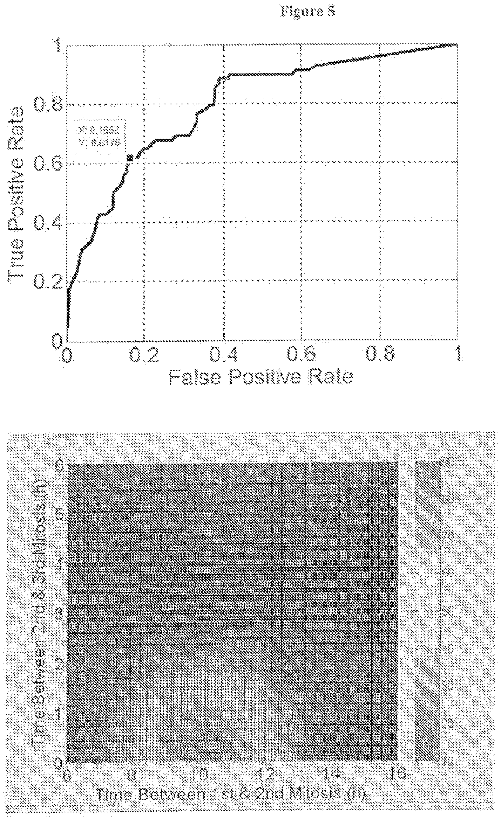

The analysis performed in Figures 4 and 5 can be leveraged to qualitatively and quantitatively inspect the development potential of each patient's cohort of embryos, for inter-embryo comparisons within a cohort, as well as inter-patient comparisons within a population. Most critically, evaluation at the cohort level reveals that, even when Eeva is in error in predicting the Usable Blastocyst (i.e., the Usable Blastocyst falls outside of the Eeva prediction window depicted in yellow), a significant majority of patients (80/95=84%) have at least one Eeva-predicted blastocyst available for selection. **Correlation with Implantation and Pregnancy Outcomes** We performed a secondary analysis to examine if the time-lapse markers used by Eeva correlate with implantation and pregnancy outcomes. Importantly, as this study was a blastocyst prediction validation study, embryos were transferred at the blastocyst stage using the standard procedures of participating clinics, and Eeva predictions were not made available at time of transfer. We observe that, of 141 embryos transferred at the blastocyst stage, those with both P2 and P3 markers within range (Eeva High) had a statistically higher chance of implantation than embryos with P2 or P3 out of range (Eeva Low) (49% vs. 21%, p<0.001) (Table 4). Similarly, for these 77 patients, those with at least one Eeva High embryo transferred were more likely to achieve clinical pregnancy (60% vs. 40%) and ongoing pregnancy (56% vs. 37%) than those with only Eeva Low embryos transferred.

Table 4

| Patient Population | # Patients | # Embryos | Age (years) | Implantation Rate | Clinical Pregnancy Rate | Ongoing Pregnancy Rate |
|---|---|---|---|---|---|---|
| At least 1 Eeva High transferred | 47 | 89 | 32.1 ± 5.2 | 49% (44/89) | 60% (28/47) | 55% (26/47) |
| Only Eeva Lows transferred | 30 | 52 | 32.2 ± 5.1 | 21% (11/52) | 40% (12/30) | 37% (11/30) |
| p-value | | | p=0.9 | p<0.001 | p=0.09 | p=0.11 |

**Overall Eeva Performance**

The overall performance of Eeva was assessed statistically by comparing predictions to the actual Usable Blastocyst outcome from the IVF clinics. In the Development phase, the Eeva prediction and cell tracking software was demonstrated to correctly predict by Day 2 those embryos which became Usable Blastocysts with a specificity of 84.2% (95% CI of 78.7% to 88.5%), sensitivity of 58.8% (95% CI of 47.0% to 69.7%), PPV of 54.1% (95% CI of 42.8% to 64.9%) and NPV of 86.6% (95% CI of 81.3% to 90.6%). In the Validation phase, the Eeva prediction and cell tracking software could correctly predict by Day 2 those embryos which became Usable Blastocysts with a specificity of 84.7% (95% CI of 81.7% to 87.3%), sensitivity of 38.0% (95% CI of 32.7% to 43.5%), PPV of 54.7% (95% CI of 48.0% to 61.2%) and NPV of 73.7% (95% CI of 70.4% to 76.8%).

As a baseline control, five clinical embryologists from five IVF clinical sites, separate from those used to manually measure parameters from the embryo videos, reviewed Day 3 morphology data for n=343 embryos. The clinical embryologists made a blinded, independent prediction about whether each embryo would become a blastocyst based on Day 3 morphology only. Morphology-based methods correctly identified those embryos which became Usable Blastocysts with a specificity of 57.0% (95% CI of 51.2% to 62.7%), sensitivity of 80.8% (95% CI of 70.7% to 87.9%), PPV of 33.7% (95% CI of 27.0% to 41.1%) and NPV of 92.3% (95% CI of 87.2% to 95.3%).

Importantly, using Eeva to predict Usable Blastocysts, the specificity and PPV were significantly improved over the average blastocyst predictions made by experienced embryologists using Day 3 morphology only (p<0.0001 and p<0.0001 for specificity and PPV using Student's T-test). Compared to the morphology approach (specificity 57.0%, PPV 33.7%), the prediction results for Eeva remained significantly improved across Development (specificity 84.2%, PPV 54.1%) and Validation datasets (specificity 84.7%, 54.7%) (**Figure 11**).

Researchers have demonstrated a benefit of time-lapse imaging in the reduced handling and removal of embryos from an optimal incubation environment (Cruz *et al.,* 2011; Kirkegaard *et al.,* 2012). Importantly, these studies have proven that time-lapse imaging is safe for continuously imaging preimplantation human embryos, causing no detrimental effect on the quality (Lemmen *et al.,* 2008; Nakahara *et al.,* 2010), developmental kinetics (Barlow *et al.,* 1992; Grisart *et al.,* 1994; Gonzales *et al.,* 1995; Kirkegaard *et al.,* 2012), blastocyst formation rate (Grisart *et al.,* 1994; Gonzales *et al.,* 1995; Pribenszky *et al.,* 2010; Cruz *et al.,* 2011; Kirkegaard *et al.,* 2012), fertilization rate (Payne *et al.,* 1997; Nakahara *et al.,* 2010), implantation rate (Kirkegaard *et al.,* 2012), pregnancy rate (Barlow *et al.,* 1992; Mio and Maeda, 2008; Cruz *et al.,* 2011; Kirkegaard *et al.,* 2012) or gene expression of human embryos (Wong *et al.,* 2010). Indeed the Eeva system operates under low power darkfield illumination that minimizes light exposure to embryos to approximately 21 seconds of what embryos experience under a conventional assisted reproduction microscope. To confirm that these culture conditions were conducive to proper embryo growth, we evaluated the overall blastocyst formation rate for patients who had blastocyst transfers in the study, and determined that the average blastocyst formation rate was 49.9%, with a range of 16.9-60.0% across sites. These values are similar to the average (45.4%) and range (28.0-60.3%) of blastocyst formation rates reported between 1998 and 2006 (Blake *et al.,* 2007), suggesting that embryos imaged by Eeva have competence for normal development.

Despite powerful observations possible with time-lapse imaging, and its confirmed safety, few studies have validated the correlation between image parameters and developmental outcomes on large sample sizes of independent data. Further, challenges in human embryo research have limited the opportunities to achieve mechanistic understanding of promising image biomarkers. Among a number of foundational studies that reported the first time-lapse observations of human embryos, including those described previously and others (Payne *et al.,* 1997; Mio and Maeda, 2008), Wong et al. described the first report of directly measureable, non-overlapping, quantitative parameters that can be readily applied to categorize embryos based on their developmental potential and intrinsic gene expression profiles. Their results demonstrated not only that the time periods of the first two cleavage divisions were predictive of success or failure to blastocyst formation, but that these durations are associated with molecular changes indicating degradation of maternal mRNAs and activation of the embryonic genome. Therefore, in this clinical study, we aimed to systematically validate the predictive power and real-time clinical utility of the cell division timings in multiple clinical settings, using Usable Blastocysts as the outcome.

We first observed that embryos that developed to blastocysts in clinical IVF settings could be predicted at the cleavage stage with similar cell division timings to previous reports. Measurement data from 292 embryos and 43 patients were used in a statistical classification tree analysis against the embryos' blastocyst formation outcomes obtained from the clinical sites. The predictor variables for Usable Blastocysts were cell division time parameters in good alignment with the timing durations previously published for cryopreserved embryos, particularly for P2 (the time between 1st and 2nd cytokinesis) and P3 (the time between 2nd and 3rd cytokinesis). Compared to the originally reported range for PI, the timing duration of P1 (duration of 1st cytokinesis) broadened in the clinical dataset, but still fell within a relatively narrow average time range of approximately 30 minutes. Thus, as expected, the discoveries of Wong et al. using cryopreserved supernumerary embryos could be extended to fresh IVF human embryos cultured to the blastocyst stage. This result is not surprising since gene expression profiling of single blastomeres and whole embryos indicated that cell division timing parameters from the 1- to 4-cell stage were linked to the transcriptional activity and molecular health of the embryos (Wong *et al.,* 2010). Together with the clinical results from sites using diverse culture protocols, the science underpinning these predictive parameters give confidence that time-lapse assessment of these key embryo developmental events may add value to current embryo selection techniques.

To build the statistical model for predicting Usable Blastocysts several statistical approaches including classification trees, linear and quadratic discriminant analysis, and Naive Bayes models were assessed, along with the inclusion of additional factors (embryo age, cell number, and method of insemination) to these models. Ultimately, the Eeva prediction and cell tracking software was based on a simple classification tree incorporating the time between 1st and 2nd cytokinesis (P2), and the time between 2nd and 3rd cytokinesis (P3). Although the duration of 1st cytokinesis (P1) was a predictor of blastocyst outcome on its own, and represents a biologically relevant step in the division of the first embryo, P2 and P3 were found to statistically dominate P1 in the prediction model. Usable Blastocyst was an important outcome of embryo competence for this study. Although selection and transfer of embryos is commonly performed following assessment on Day 3, blastocyst transfer on Day 5 or Day 6 is gaining favor (Gardner *et al.,* 2000)(Diamond *et al.,* 2012). Blastocyst transfer selects embryos which progress successfully to the blastocyst stage, and has been shown to result in close to twice the implantation rates of Day 3 transfer (Papanikolaou *et al.,* 2005; Papanikolaou *et al.,* 2006; Blake *et al.,* 2007;) (Gelbaya *et al.,* 2010). However, for many patients and laboratories, there are disadvantages and risks associated with the practice of blastocyst transfer. Nearly half of embryos that appear to be of good quality have been reported to arrest over prolonged culture from the cleavage to blastocyst stage (Niemitz and Feinberg, 2004; Horsthemke and Ludwig, 2005; Manipalviratn *et al.,* 2009). Consequently, blastocyst transfer is often avoided, particularly for poor prognosis patients who have only few embryos that may fail to survive extended culture conditions. In addition, it has been suggested that prolonged culture can increase the risk of epigenetic disorders, monozygotic twinning and associated complications, pregnancy complications such as preterm delivery and low birth weight, and long-term health issues for offspring of assisted reproduction (Milki *et al.,* 2003; Niemitz and Feinberg., 2004; Horsthemke and Ludwig, 2005; Manipalviratn *et al.,* 2009; Kallen *et al.,* 2010; Kalra *et al.,* 2012). Identification of blastocysts by the cleavage stage of development may reduce the need to perform extended culture for selection purposes (Coskun *et al.,* 2000; Milki *et al.,* 2002) and enable early transfer of a single embryo. In turn, earlier transfer practices may positively impact lab workflow conditions, reduce costs associated with embryo culture, as well as potentially improve the health of the embryo. Interestingly, in our patient population, 4 out of 7 of the patients who had no blastocysts on Day 5 had at least one embryo that was predicted to become a Usable Blastocyst based on Eeva's prediction (see **Figures 10** and **11**). It is conceivable that Day 3 transfer of these predicted blastocysts would have prevented their arrest and resulted in favorable implantation outcomes, although additional studies are needed to directly address this hypothesis.

Clinical adoption of new embryo selection technology depends not only on the scientific and clinical merit of its predictive parameters, but also on how the technology fits in the fast-paced and high volume workflow of the IVF laboratory. Time-lapse image parameters, also referred to as "morphokinetics", may be manually extracted from time-lapse images, but it is a time-consuming and laborious process prone to observer variability (Baxter Bendus *et al.,* 2006). We observed that on average, it took approximately 3 hours for a highly experienced embryologist to review embryo movies and measure a few specific cell division timing parameters for 25 embryos (~7 minutes per embryo). In contrast, in routine clinical practice it is typical for only 15-30 minutes to be allotted for an embryologist to assess embryos and prepare a case for transfer. Thus, to enable rapid, quantitative and reproducible assessment of time-lapse parameters in clinical settings, we developed cell tracking software that automatically tracks cell shape, location, and division over time. While there have been a few recent technical reports on automated image analysis of human embryo microscope images (Filho *et al.,* 2010) (Filho *et al.,* 2012), to our knowledge, there has been only a single successful demonstration of image analysis software applied to time-lapse imaging of human embryo development (Wong *et al.,* 2010). Automated image analysis of time-lapse videos is particularly challenging due to the abundance of data that must be processed over time.

In the present work, we extended the cell tracking framework introduced in Wong et al. to develop software that evaluates a series of human embryo images, directly detects cell membranes, identifies the timing of the divisions from the 1-cell stage to the 4-cell stage, and generates predictions of embryo development for all embryos in a dish in real-time. We then validated the tracking and prediction accuracy of the Eeva software on an independent dataset of embryos for which blastocyst outcomes were blinded. Eeva software measurements had very high (>90%) agreement with manual measurements, and disagreed in cases where embryos exhibited complex dynamic behaviors that were also difficult to manually assess - in many of these cases, the embryologists displayed a high degree of inter-observer variability in their expert review (data not shown). Overall, the sophisticated image analysis and cell tracking software improved measurement objectivity, consistency and efficiency compared to subjective assessment by human observers.

In a final and blinded test of Eeva's performance, we applied Eeva's integrated prediction and cell tracking capabilities to an independent Validation Dataset and compared the predictions generated by Eeva to those generated by skilled embryologists using Day 3 morphological criteria. The specificity of Usable Blastocyst prediction was significantly improved when using Eeva compared to morphology (84.7% vs. 54.7%, p<0.0001). Importantly, the Eeva prediction model was designed to optimize specificity out of consideration that the main limitation in traditional morphology is its high sensitivity and low specificity, or its tendency to deem most "good morphology" embryos as viable. Clinical results have shown that many embryos selected on the basis of good morphology criteria alone are false positives that do not form blastocysts and do not implant. The IVF field is thus in need of a test which can help discriminate - among the embryos with good morphology - those which will form viable blastocysts with highest developmental potential. In the current study, Eeva's specificity indicated that of all arrested embryos, Eeva could correctly identify 84.7% of them as "poor" or "low probability" to form blastocysts, while Morphology could only correctly identify 54.7% of them as "poor" (p<0.0001). The specificity also indicated that Eeva reduced the false positive rates commonly associated with Morphology-based selection from 43.0% to 15.3%.

The substantial benefit of Eeva is in its ability to provide quantitative information to clinicians that improves embryo selection accuracy by significantly improving specificity and thereby reducing false positive rates. The results of the independent validation also determined the positive predictive value of blastocyst prediction, or ability to correctly identify blastocysts, to be significantly improved from 33.7% using Morphology to 54.7% using Eeva (p<0.0001). However, both the sensitivity and negative predictive value decreased with Eeva. Preliminary observations suggest that false negatives associated with the low sensitivity (predicted to arrest but actually developed to blastocysts) may be indicative of blastocysts that have lower implantation potential. An ongoing study is evaluating the contribution of this high specificity technology to embryo implantation.

Results from this study take into account different stimulation protocols, fertilization methods, embryo culture media, and incubation conditions, as each of the five participating IVF clinics followed their own protocols throughout the IVF procedure. We performed a sub-analysis of the specificity and sensitivity for Eeva as a function of clinical site, fertilization method, and egg age and found no statistical difference among each group (data not shown). Further, we developed the Eeva prediction model on a relatively broad and representative patient population designated to Day 3 or Day 5 transfer, despite requiring relatively good prognosis patients in the Validation Dataset to test the model for blastocyst outcome. These findings suggest that the high 85% specificity of Eeva assessment may be widely applicable and provide useful information that can impact embryo selection for many users across clinical sites and protocols.

Evidence-based validation of clinical usefulness is essential before implementing new diagnostic tools in IVF laboratories. This is the first early embryo viability assessment approach that integrates time-lapse imaging with (1) predictive parameters rooted in the underlying molecular physiology of embryos, (2) automated cell tracking software, and (3) successful clinical validation. Here, validation was achieved in a steady and step-wise fashion that extended our original scientific report to a prospectively designed study testing the positive and negative predictive values of the parameters, as well as the accuracy of the first cell tracking software tool designed to automate time-lapse parameter measurements.

Overall, the results of the present study demonstrate that Eeva can be safely and easily implemented in the lab with discernible results in an overwhelming number (94.2%) of embryos, yielding consistent, real-time predictions of embryo viability with significantly improved specificity, PPV and overall accuracy over morphology.

## EXAMPLE 4

To improve the embryo selection process, additional assessment parameters are needed by de-selecting from that group of good morphology embryos those that have a low likelihood to become blastocysts. The high specificity of the blastocyst prediction model can be leveraged to address this known limitation in traditional morphology, and help the embryologist identify those embryos with good Day 3 morphology that have a Low Probability to become blastocysts.

Three clinical embryology laboratory directors (separate from the observer panel used to develop and test the Eeva prediction model) reviewed data in two independent sessions that assessed their prediction of usable blastocyst formation. During the first prediction session, embryologists were given D3 morphology (SART) data, including: number of cells, fragmentation (0%, <10%, 11-25%, >25%), symmetry (perfect, moderately asymmetrical, severely asymmetrical), and age of patient or egg donor. Each embryologist was blinded to the predictions of other embryologists. One week later, during a second prediction session, the same embryologists were given D3 morphology (SART) data as above and Eeva data for the same embryos. In this session, each embryologist was blinded to the predictions of other embryologists and the predictions from the first session. Eeva data included the cell cycle parameter values (P2 and P3) and a prediction score of "high" or "low" probability of usable blastocyst formation, based on the classification tree cutoffs determined in the Development Phase. To quantify the embryo selection performance of the two methods, predictions made in each session (using morphology only or morphology plus Eeva) were compared to the usable blastocyst outcome.

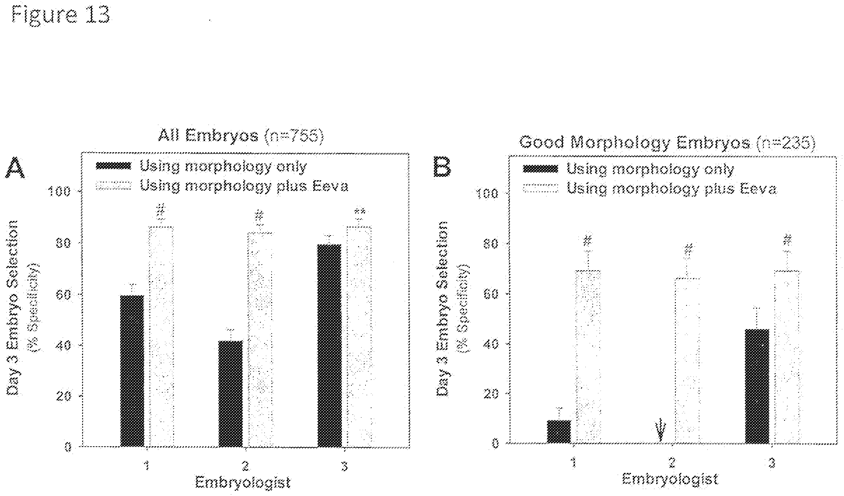

The utility of combining Eeva with traditional morphology assessment for D3 embryo selection was examined using a sub-analysis of patients with full cohorts of D5 embryos. Using D3 morphology only, embryologists 1, 2, and 3 selected embryos with a baseline specificity of 59.7%, 41.9%, and 79.5% and a baseline PPV of 45.5%, 41.5%, and 50.5%. When Eeva information was added to morphology on D3, each embryologist improved their selection of usable blastocysts to a specificity of 86.3% (p<0.0001), 84.0% (p<0.0001), and 86.6% (p<0.01) (**Figure 13A**) and a PPV of 56.3% (p<0.05), 52.1% (p<0.05), and 55.5% (p=0.34). The improvement for all embryologists was also accompanied by a reduction in variability among embryologists. Using D3 morphology alone, there was a 37.7% maximum difference in specificity and 8.9% maximum difference in PPV among embryologists. In contrast, using D3 morphology plus Eeva, there was a 2.5% maximum difference in specificity and 4.2% difference in PPV.

Because standard morphological grading can identify "good" morphology embryos, we assessed whether Eeva could help embryologists discriminate on D3 which "good" morphology embryos would most likely develop to the usable blastocyst stage. For this analysis, embryos with "good" morphology were defined as having 6- to 10-cells, <10% fragmentation, and perfect symmetry. Using morphology only, embryologists 1, 2, and 3 varied considerably in their selection of which "good" embryos would become usable blastocysts (specificity 9.0%, 0.0%, and 45.9%, respectively). Using morphology plus Eeva, each embryologist improved their D3 selection to a specificity of 69.2% (p<0.0001), 66.2% (p<0.0001), and 69.2% (p<0.01), respectively (**Figure 13B**). For embryos with "poor" morphological criteria on D3, the selections of all embryologists were also improved (specificity: 77.5% vs. 92.3%, p<0.0001 for embryologist 1; 56.5% vs. 90.3%, p<0.0001 for embryologist 2; 91.3% vs. 92.6%, p=0.54 for embryologist 3). These data show that, combined with D3 morphological assessment, Eeva provides valuable information to help embryologists identify which embryos that are favored by morphology are likely to subsequently arrest.

Table 5 below summarizes a particular recommendations on how to combine the model results with traditional morphology for the Adjunct Prediction.

**Table 5. Recommendations for Adjunct Prediction**

| When blastocyst prediction model and morphology are in agreement: | Recommendation |
|---|---|
| When embryo morphology = 'Good' or 'Fair', and Model = 'High Probability Blastocyst'; - **OR**-When embryo morphology = 'Poor' and Model = 'Low Probability Blastocyst' | Follow the combined recommendation |
| When blastocyst prediction model and morphology are in disagreement: | Recommendation |
| When embryo morphology = 'Good' or 'Fair', and Model = 'Low Probability for Blastocyst' | Favor the Model - embryo has low likelihood to become a blastocyst |
| When embryo morphology = 'Poor', and Model = 'High Probability for B lastocyst' | Favor morphology - embryo has low likelihood to be viable |

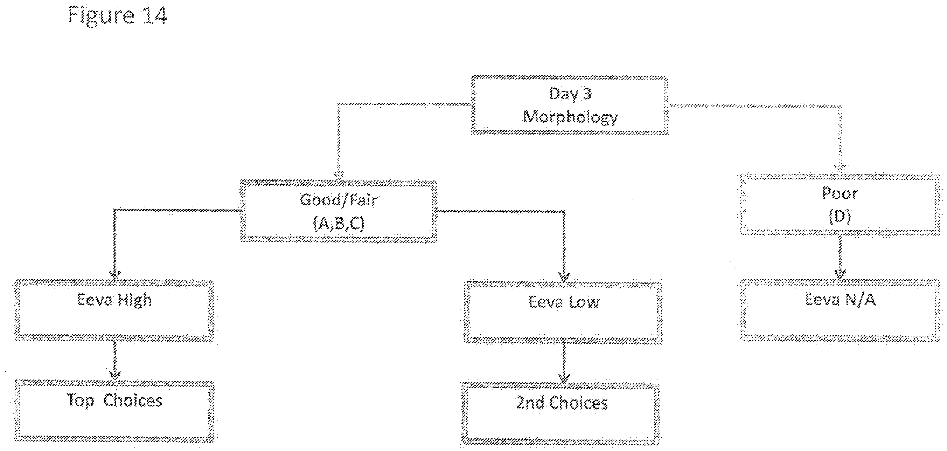

Alternatively, an embryologist may take a sequential approach to the use of morphology and information on the events occurring during the first two days of development. A schematic of the "sequential approach" is depicted in Figure 14.

This approach is particularly powerful in that we observed that by using morphology and Eeva sequentially, embryologists are three times more likely to detect a true

Table 6

| N=54 (patients) | | Morph. Alone % | Sequential % | P Value |
|---|---|---|---|---|
| A,B, C Embryos (Remove D*) | Sensitivity | 85.6%** | 40.4% | P<0.001 |
| | Specificity | 25.7%** | 76.0% | P<0.001 |

| (n=500) | | | | |
|---|---|---|---|---|

*Excludes 258 embryos as a result of poor morphology

** At this stage, morphology is of little use for further selection.

When analyzing embryos that received an A, B or C grade based on morphology, using a follow on Eeva prediction, we were able to predict blastocyst formation in 56% of embryos. This is significant since the overall blastocyst prevalence of A,B,C embryos without using sequential Eeva adjunct prediction is only 42%. Therefore, by selecting Eeva high embryos, an embryologist increases the likelihood of a true positive by 14% relative to the overall blast prevalence in the A, B, C embryo population. (Table 7)

Table 7

| N=54 (patients) | Eeva Prediction | % Blast (CI) | P Value |
|---|---|---|---|
| A,B, C embryos (n=500*) | High | 56% (48%-64%) | P<0.001 |
| | Low | 36% (31-41%) | P<0.001 |

*Blast prevalence in population =42%

A demonstration of clinical utility is essential before any new tool is introduced into IVF laboratories. Therefore, an Adjunct Assessment sub-analysis was conducted to assess whether adding automated Eeva predictions to traditional morphological methods could aid experienced embryologists in D3 embryo selection.

Results demonstrated that when Eeva was used in combination with D3 morphology, embryologists experienced significant improvement in the likelihood of selecting embryos that would develop to usable blastocysts. In particular, combining the high specificity of Eeva with traditional morphology methods dramatically improved the ability to determine the developmental potential of "good" morphology embryos. Notably, there is strikingly high variability in the morphology-based selections of embryologists reviewing "good" embryos, as their specificities spanned from 0% (because one embryologist considered that all of these embryos would develop to usable blastocyst) to 45.9% (because of the less conservative approach of another embryologist).

Using morphology plus Eeva, the average of the three embryologists' prediction specificities were significantly improved (68.2 $\pm$ 1.7% for morphology plus Eeva vs. 18.3 $\pm$ 23.3% for morphology alone, p<0.05). The embryologists' performances were also more consistent, as the standard deviation among embryologists was reduced. It is widely accepted that morphological grading is accompanied by significant intra- and inter-operator variability which can impact IVF success rates (Baxter Benus, 2006; Paternot, 2009). Here, we have built a generalized prediction algorithm based on multi-clinic data and demonstrated that the automated prediction information can be added to embryologists' morphological evaluations to improve their inter-operator variability. Combining the non-invasive, automated Eeva measurements with traditional morphology will provide embryologists with more consistent and objective data that may make embryo assessment on D3 more standardized, reproducible and successful.

**REFERENCES**

Alpha E. The Istanbul consensus workshop on embryo assessment: proceedings of an expert meeting. Hum Reprod 2011;26:1270-1283.

Assidi M, Montag M, Van der Ven K, Sirard MA. Biomarkers of human oocyte developmental competence expressed in cumulus cells before ICSI: a preliminary study. J Assist Reprod Genet 2011;28:173-188.

Barlow P, Puissant F, Van der Zwalmen P, Vandromme J, Trigaux P, Leroy F. In vitro fertilization, development, and implantation after exposure of mature mouse oocytes to visible light. Mol Reprod Dev 1992;33:297-302.

Baxter Bendus AE, Mayer JF, Shipley SK, Catherino WH. Interobserver and intraobserver variation in day 3 embryo grading. Fertil Steril 2006;86:1608-1615.

Blake DA, Farquhar CM, Johnson N, Proctor M. Cleavage stage versus blastocyst stage embryo transfer in assisted conception. Cochrane Database Syst Rev 2007;CD002118.

Chen AA, Tan L, Suraj V, Reijo Pera R, Shen S, Biomarkers identified with time-lapse imaging: discovery, validation, and practical application, Fertil. Steril 2013; 99:1035-43.

Coskun S, Hollanders J, Al-Hassan S, Al-Sufyan H, Al-Mayman H, Jaroudi K. Day 5 versus day 3 embryo transfer: a controlled randomized trial. Hum Reprod 2000;15:1947-1952.

Cruz M, Gadea B, Garrido N, Pedersen KS, Martinez M, Perez-Cano I, Munoz M, Meseguer M. Embryo quality, blastocyst and ongoing pregnancy rates in oocyte donation patients whose embryos were monitored by time-lapse imaging. J Assist Reprod Genet 2011;

Cruz, M, Garrido N, Herrero J, Perez-Cano, I, Munoz M, Meseguer M. Timing of cell division in human cleavage-stage embryos is linked with blastocyst formation and quality. Reprod. Biomed. Online 2012;25:371-81.

De Placido G, Wilding M, Strina I, Alviggi E, Alviggi C, Mollo A, Varicchio MT, Tolino A, Schiattarella C, Dale B. High outcome predictability after IVF using a combined score for zygote and embryo morphology and growth rate. Hum Reprod 2002;17:2402-2409.

Desai NN, Goldstein J, Rowland DY, Goldfarb JM (2000) Morphological evaluations of human embryos and derivation of an embryo quality scornig system specific for day 3 embryos: a preliminary study. Human Reprod 2000;15:2190-2196

Diamond MP, Willman S, Chenette P, Cedars MI. The clinical need for a method of identification of embryos destined to become a blastocyst in assisted reproductive technology cycles. J Assist Reprod Genet 2012;29:391-396.

Fenwick J, Platteau P, Murdoch AP, Herbert M. Time from insemination to first cleavage predicts developmental competence of human preimplantation embryos in vitro. Hum Reprod 2002;17:407-412.

Filho ES, Noble JA, Poli M, Griffiths T, Emerson G, Wells D. A method for semi-automatic grading of human blastocyst microscope images. Hum Reprod 2012;

Filho ES, Noble JA, Wells D. A review on automatic analysis of human embryo microscope images. Open Biomed Eng J 2010;4:170-177.

Fragouli E, Wells D. Aneuploidy screening for embryo selection. Semin Reprod Med 2012;30:289-301.

Gardner DK, Lane M, Stevens J, Schlenker T, Schoolcraft WB. Blastocyst score affects implantation and pregnancy outcome: towards a single blastocyst transfer. Fertil Steril 2000;73:1155-1158.

Gelbaya TA, Tsoumpou I, Nardo LG. The likelihood of live birth and multiple birth after single versus double embryo transfer at the cleavage stage: a systematic review and meta-analysis. Fertil Steril 2010;94:936-945.

Gonzales DS, Pinheiro JC, Bavister BD. Prediction of the developmental potential of hamster embryos in vitro by precise timing of the third cell cycle. J Reprod Fertil 1995;105:1-8.

Grisart B, Massip A, Dessy F. Cinematographic analysis of bovine embryo development in serum-free oviduct-conditioned medium. J Reprod Fertil 1994;101:257-264.

Guerif F, Lemseffer M, Leger J, Bidault R, Cadoret V, Chavez C, Gasnier O, Saussereau MH, Royere D. Does early morphology provide additional selection power to blastocyst selection for transfer? Reprod Biomed Online 2010;21:510-519.

Hardarson T, Hanson C, Sjogren A, Lundin K. Human embryos with unevenly sized blastomeres have lower pregnancy and implantation rates: indications for aneuploidy and multinucleation. Hum Reprod 2001;16:313-318.

Hashimoto S, Kato N, Saeki K, Morimoto Y. Selection of high-potential embryos by culture in poly(dimethylsiloxane) microwells and time-lapse imaging. Fertil Steril 2012;97:332-337.

Horsthemke B, Ludwig M. Assisted reproduction: the epigenetic perspective. Hum Reprod Update 2005;11:473-482.

Kallen B, Finnstrom O, Lindam A, Nilsson E, Nygren KG, Otterblad Olausson P. Trends in delivery and neonatal outcome after in vitro fertilization in Sweden: data for 25 years. Hum Reprod 2010;25:1026-1034.

Kalra SK, Ratcliffe SJ, Barnhart KT, Coutifaris C. Extended Embryo Culture and an Increased Risk of Preterm Delivery. Obstetrics & Gynecology 2012;120:69-75 10.1097/AOG.1090b1013e31825b31888fc.

Kirkegaard K, Agerholm IE, Ingerslev HJ. Time-lapse monitoring as a tool for clinical embryo assessment. Hum Reprod 2012a;27:1277-1285.

Kirkegaard K, Hindkjaer JJ, Grondahl ML, Kesmodel US, Ingerslev HJ. A randomized clinical trial comparing embryo culture in a conventional incubator with a time-lapse incubator. J Assist Reprod Genet 2012b;

Lemmen JG, Agerholm I, Ziebe S. Kinetic markers of human embryo quality using time-lapse recordings of IVF/ICSI-fertilized oocytes. Reprod Biomed Online 2008;17:385-391.

Lundin K, Bergh C, Hardarson T. Early embryo cleavage is a strong indicator of embryo quality in human IVF. Hum Reprod 2001;16:2652-2657.

Machiter R and Racowsky C. Morphological systems of human embryo assesment and clinical evidence. Reproductive Bio Medicine Online 2013;26:210-221

Manipalviratn S, DeCherney A, Segars J. Imprinting disorders and assisted reproductive technology. Fertil Steril 2009;91:305-315.

Meseguer M, Herrero J, Tejera A, Hilligsoe KM, Ramsing NB, Remohi J. The use of morphokinetics as a predictor of embryo implantation. Hum Reprod 2011;26:2658-71Milki AA, Hinckley MD, Behr B. Comparison of blastocyst transfer to day 3 transfer with assisted hatching in the older patient. Fertil Steril 2002;78:1244-1247.

Milki AA, Jun SH, Hinckley MD, Behr B, Giudice LC, Westphal LM. Incidence of monozygotic twinning with blastocyst transfer compared to cleavage-stage transfer. Fertil Steril 2003;79:503-506.

Mio Y, Maeda K. Time-lapse cinematography of dynamic changes occurring during in vitro development of human embryos. Am J Obstet Gynecol 2008;199:660 e661-665.

Montag M, Liebenthron J, Koster M. Which morphological scoring system is relevant in human embryo development? Placenta 2011;32:S252-S256.

Nakahara T, Iwase A, Goto M, Harata T, Suzuki M, Ienaga M, Kobayashi H, Takikawa S, Manabe S, Kikkawa F, et al. Evaluation of the safety of time-lapse observations for human embryos. J Assist Reprod Genet 2010;27:93-96.

Nel-Themaat L, Nagy ZP. A review of the promises and pitfalls of oocyte and embryo metabolomics. Placenta 2011;32 Suppl 3:S257-263.

Niemitz EL, Feinberg AP. Epigenetics and assisted reproductive technology: a call for investigation. Am J Hum Genet 2004;74:599-609.

Papanikolaou EG, Camus M, Kolibianakis EM, Van Landuyt L, Van Steirteghem A, Devroey P. In vitro fertilization with single blastocyst-stage versus single cleavage-stage embryos. N Engl J Med 2006;354:1139-1146.

Papanikolaou EG, D'Haeseleer E, Verheyen G, Van de Velde H, Camus M, Van Steirteghem A, Devroey P, Tournaye H. Live birth rate is significantly higher after blastocyst transfer than after cleavage-stage embryo transfer when at least four embryos are available on day 3 of embryo culture. A randomized prospective study. Hum Reprod 2005;20:3198-3203.

Patemot G, Deroe J, Debrock S, D'Hooghe TM, Spiessens C. Intra-and Inter-observer analysis in the morpholigical assessment of early-stage embryos. Reproductive biology and endocrinology: RB&E 2009;7:105.

Payne D, Flaherty SP, Barry MF, Matthews CD. Preliminary observations on polar body extrusion and pronuclear formation in human oocytes using time-lapse video cinematography. Hum Reprod 1997;12:532-541.

Pribenszky C, Losonczi E, Molnar M, Lang Z, Matyas S, Rajczy K, Molnar K, Kovacs P, Nagy P, Conceicao J, et al. Prediction of in-vitro developmental competence of early cleavage-stage mouse embryos with compact time-lapse equipment. Reprod Biomed Online 2010;20:371-379.

Racowsky C, Vernon M, Mayer J, Ball GD, Behr B, Pomeroy KO, Wininger D, Gibbons W, Conaghan J, Stern JE. Standardization of grading embryo morphology. Fertil Steril 2010;94:1152-1153.

Rijnders PM, Jansen CA. Influence of group culture and culture volume on the formation of human blastocysts: a prospective randomized study. Hum Reprod 1999;14:2333-2337.

Scott R, Tao X, Taylor D, Ferry KM, Treff NR. A prospective randomized controlled trial demonstrating significantly increased clinical pregnancy rates following 24 chromosome aneuploidy screening: biopsy and analysis on day 5 with fresh transfer. Fertil Steril 2010;94:S2.

Scott RT, Jr., Ferry K, Su J, Tao X, Scott K, Treff NR. Comprehensive chromosome screening is highly predictive of the reproductive potential of human embryos: a prospective, blinded, nonselection study. Fertil Steril 2012;97:870-875.

Seli E, Robert C, Sirard MA. OMICS in assisted reproduction: possibilities and pitfalls. Mol Hum Reprod 2010a;16:513-530.

Seli E, Vergouw CG, Morita H, Botros L, Roos P, Lambalk CB, Yamashita N, Kato O, Sakkas D. Noninvasive metabolomic profiling as an adjunct to morphology for noninvasive embryo assessment in women undergoing single embryo transfer. Fertil Steril 2010b;94:535-542.

Sugimura S, Akai T, Somfai T, Hirayama M, Aikawa Y, Ohtake M, Hattori H, Kobayashi S, Hashiyada Y, Konishi K, et al. Time-lapse cinematography-compatible polystyrene-based microwell culture system: a novel tool for tracking the development of individual bovine embryos. Biol Reprod 2010;83:970-978.

Swann K, Windsor S, Campbell K, Elgmati K, Nomikos M, Zernicka-Goetz M, Amso N, Lai FA, Thomas A, Graham C. Phospholipase C-zeta-induced Ca2+ oscillations cause coincident cytoplasmic movements in human oocytes that failed to fertilize after intracytoplasmic sperm injection. Fertil Steril 2012;97:742-747.

Vajta G, Peura TT, Holm P, Paldi A, Greve T, Trounson AO, Callesen H. New method for culture of zona-included or zona-free embryos: the Well of the Well (WOW) system. Mol Reprod Dev 2000;55:256-264.

van Montfoort AP, Dumoulin JC, Land JA, Coonen E, Derhaag JG, Evers JL. Elective single embryo transfer (eSET) policy in the first three IVF/ICSI treatment cycles. Hum Reprod 2005;20:433-436.

Vergouw CG, Kieslinger DC, Kostelijk EH, Botros LL, Schats R, Hompes PG, Sakkas D, Lambalk CB. Day 3 embryo selection by metabolomic profiling of culture medium with near-infrared spectroscopy as an adjunct to morphology: a randomized controlled trial. Hum Reprod 2012;

Vernon M, Stern JE, Ball GD, Wininger D, Mayer J, Racowsky C. Utility of the national embryo morphology data collection by the Society for Assisted Reproductive Technologies (SART): correlation between day-3 morphology grade and live-birth outcome. Fertil Steril 2011;95:2761-2763.

Wathlet S, Adriaenssens T, Segers I, Verheyen G, Janssens R, Coucke W, Devroey P, Smitz J. New candidate genes to predict pregnancy outcome in single embryo transfer cycles when using cumulus cell gene expression. Fertil Steril 2012;

Wathlet S, Adriaenssens T, Segers I, Verheyen G, Van de Velde H, Coucke W, Ron El R, Devroey P, Smitz J. Cumulus cell gene expression predicts better cleavage-stage embryo or blastocyst development and pregnancy for ICSI patients. Hum Reprod 2011;26:1035-1051.

Wong C, Loewke K, Bossert N, Behr B, De Jonge C, Baer T, Reijo Pera R. Non-invasive imaging of human embryos before embryonic genome activation predicts development to the blastocyst stage. Nat Biotechnol 2010;28:1115-1121.

Yang Z, Liu J, Collins GS, Salem SA, Liu X, Lyle SS, Peck AC, Sills ES, Salem RD. Selection of single blastocysts for fresh transfer via standard morphology assessment alone and with array CGH for good prognosis IVF patients: results from a randomized pilot study. Molecular cytogenetics 2012;5:24.

The preceding merely illustrates the principles of the invention. It will be appreciated that those skilled in the art will be able to devise various arrangements which, although not explicitly described or shown herein, embody the principles of the invention and are included within its and scope. Furthermore, all examples and conditional language recited herein are principally intended to aid the reader in understanding the principles of the invention and the concepts contributed by the inventors to furthering the art, and are to be construed as being without limitation to such specifically recited examples and conditions.

Moreover, all statements herein reciting principles, aspects, and embodiments of the invention as well as specific examples thereof, are intended to encompass both structural and functional equivalents thereof. Additionally, it is intended that such equivalents include both currently known equivalents and equivalents developed in the future, i.e., any elements developed that perform the same function, regardless of structure. The scope of the present invention, therefore, is not intended to be limited to the exemplary embodiments shown and described herein. Rather, the scope of the present invention is embodied by the appended claims.

STATEMENTS OF THE DISCLOSURE

A. A high specificity method for selecting one or more human embryos that is likely to reach the blastocyst stage comprising: culturing one or more human embryos under conditions sufficient for embryo development; time lapse imaging said one or more embryos for the duration of at least one mitotic cell cycle; measuring cellular parameters comprising: (a) the time interval between mitosis 1 and mitosis 2; and (b) the time interval between mitosis 2 and mitosis 3, and selecting an embryo that is likely to reach the blastocyst stage when, (i) the time interval between the resolution of mitosis 1 and the onset of mitosis 2 is about 9.33-11.45 hours; and (ii) the time interval between the onset of mitosis 2 and the onset of mitosis 3 is about 0-1.73 hours; thereby selecting with high specificity one or more human embryos that is likely to reach the blastocyst stage.

B. The method of statement A wherein said cellular parameters are measured by time-lapse microscopy.

C. The method of statement A wherein said cellular parameters further comprise the duration of cell cycle 1.

D. The method of statement C wherein an embryo is more likely to reach the blastocyst stage when the duration of cell cycle 1 is about 20 to about 27 hours.

E. The method of statement A wherein said human embryo is not indicated to be likely to reach the blastocyst stage when there is (a) a longer or shorter time interval between the resolution of mitosis 1 and the onset of mitosis 2 for said human embryo than for said reference human embryo; or (b) a longer time interval between the initiation of mitosis 2 and the onset of mitosis 3 for said human embryo than for said reference human embryo.

F. The method of statement E wherein said cellular parameters further comprise the duration of cell cycle 1.

G. The method of statement F wherein said human embryo is not indicated to be more likely to reach the blastocyst stage when the duration of cell cycle 1 is longer than about 27 hours.

H. The method of statement A wherein said embryos are produced by fertilization of oocytes in vitro.

I. The method of statement A wherein said oocytes are matured in vitro.

J. The method of statement I wherein said oocytes matured in vitro are supplemented with growth factors.

K. The method of statement A wherein the embryos have not been frozen prior to measuring the parameters.

L. The method of statement A wherein the embryos have been frozen prior to measuring the parameters.

M. The method of statement A further comprising implanting the human embryo selected to be more likely to reach the blastocyst stage into a female human subject.

N. The method of statement A further comprising freezing the human embryo selected to be more likely to reach the blastocyst stage.

O. The method of statement A wherein the measuring the cellular parameters is automated.

P. The method of statement A wherein the selecting with high specificity one or more human embryos that is more likely to reach the blastocyst stage is automated.

Q. The method of statement A wherein said time lapse imaging acquires images that are digitally stored.

R. The method of statement A wherein said time lapse imaging employs darkfield illumination.

S. The method of statement A wherein said one or more human embryos are placed in a culture dish prior to culturing under conditions sufficient for embryo development.

T. The method of statement S wherein said culture dish comprises a plurality of microwells.

U. The method of statement T wherein said culture dish comprises from 1 to about 30 microwells.

V. The method of statement T wherein one or more human embryos is placed within a microwell prior to culturing under conditions sufficient for embryo development.

W. The method of statement A wherein the measuring is carried out at an imaging station.

X. The method of statement A wherein the one or more human embryos selected to be more likely to reach the blastocyst stage has the capacity to successfully implant into a uterus.

Y. The method of statement X wherein the one or more human embryos with the capacity to successfully implant into the uterus has the capacity to go through gestation.

Z. The method of statement Y wherein the one or more human embryos with the capacity to go through gestation has the capacity to be born live.

AA. The method of statement A wherein selecting with high specificity comprises transferring said one or more human embryos.

AB. The method of statement AA wherein said transferring is done by the 4 cell stage.

AC. A high specificity method for selecting one or more human embryos that is not likely to reach the blastocyst stage comprising: culturing one or more human embryos under conditions sufficient for embryo development; time lapse imaging said one or more embryos for the duration of at least one mitotic cell cycle; measuring cellular parameters comprising: (a) the time interval between mitosis 1 and mitosis 2; and (b) the time interval between mitosis 2 and mitosis 3, and selecting an embryo that is not likely to reach the blastocyst stage when, (i) the time interval between the resolution of mitosis 1 and the onset of mitosis 2 is less than about 9.33 hours or more than about 11.45 hours; or (ii) the time interval between the onset of mitosis 2 and the onset of mitosis 3 is greater than about 1.73 hours; thereby selecting with high specificity one or more human embryos that is not likely to reach the blastocyst stage.

AD. A high specificity method for selecting one or more human embryos that is likely to reach the usable blastocyst stage comprising: culturing one or more human embryos under conditions sufficient for embryo development; time lapse imaging said one or more embryos for the duration of at least one mitotic cell cycle; measuring cellular parameters comprising: (a) the time interval between mitosis 1 and mitosis 2; and (b) the time interval between mitosis 2 and mitosis 3, and selecting an embryo that is likely to reach the usable blastocyst stage when, (i) the time interval between the resolution of mitosis 1 and the onset of mitosis 2 is about 9.33-11.45 hours; and (ii) the time interval between the onset of mitosis 2 and the onset of mitosis 3 is about 0-1.73 hours; thereby selecting with high specificity one or more human embryos that is likely to reach the usable blastocyst stage.

AE. A high specificity method for selecting one or more human embryos that is not likely to reach the usable blastocyst stage comprising: culturing one or more human embryos under conditions sufficient for embryo development; time lapse imaging said one or more embryos for the duration of at least one mitotic cell cycle; measuring cellular parameters comprising: (a) the time interval between mitosis 1 and mitosis 2; and (b) the time interval between mitosis 2 and mitosis 3, and selecting an embryo that is not likely to reach the usable blastocyst stage when, (i) the time interval between the resolution of mitosis 1 and the onset of mitosis 2 is less than about 9.33 hours or more than about 11.45 hours; or (ii) the time interval between the onset of mitosis 2 and the onset of mitosis 3 is greater than about 1.73 hours; thereby selecting with high specificity one or more human embryos that is not likely to reach the usable blastocyst stage.

AF. A high specificity method for assessing whether one or more human embryos is likely to reach the usable blastocyst stage comprising: culturing one or more human embryos under conditions sufficient for embryo development; time lapse imaging said one or more embryos for the duration of at least one mitotic cell cycle; measuring cellular parameters comprising: (a) the time interval between mitosis 1 and mitosis 2; and (b) the time interval between mitosis 2 and mitosis 3, and determining that an embryo that is likely to reach the usable blastocyst stage when, (i) the time interval between the resolution of mitosis 1 and the onset of mitosis 2 is about 9.33-11.45 hours; and (ii) the time interval between the onset of mitosis 2 and the onset of mitosis 3 is about 0-1.73 hours; and selecting with high specificity one or more human embryos that is likely to reach the usable blastocyst stage.

AG. A method for selecting one or more human in vitro fertilized embryos that is likely to reach the blastocyst stage comprising: culturing one or more human embryos in vitro under conditions sufficient for embryo development; time lapse imaging said one or more human embryos; determining whether an embryo is of good or poor quality by morphological assessment; measuring cellular parameters comprising: (a) the time interval between mitosis 1 and mitosis 2; and (b) the time interval between mitosis 2 and mitosis; 3 and selecting an embryo that is likely to reach the blastocyst stage when: the morphological assessment determines the embryo is of good quality and the time interval between mitosis 1 and mitosis 2 is about 9.33-11.45 hours; and the time interval between mitosis 2 and mitosis 3 is about 0-1.73 hours.

AH. The method of statement AG wherein the morphological assessment is done before, concurrently with or after the measurement of the cellular parameters.

AI. The method of statement AG wherein the morphological assessment is done at day 3 post insemination.

AJ. The method of statement AI wherein the morphological assessment includes determining the number of cells, determining the level of fragmentation and/or determining the symmetry of the blastomeres.

AK. The method of statement AJ wherein the morphological assessment determines an embryo is of good quality when the embryo has 6-10 cells, has less than 10% fragmentation and has symmetrical blastomeres.

AL. The method of statement AG further comprising implanting the human embryo selected to be more likely to reach the blastocyst stage into a female subject.

AM. The method of statement AG further comprising freezing the human embryo selected to be more likely to reach the blastocyst stage.

AN. The method of statement AG wherein the measuring of the cellular parameters is automated.

AO. The method of statement AG wherein said one or more human embryos are placed in a culture dish prior to culturing under conditions sufficient for embryo development.

AP. The method of statement AG wherein the one or more human embryos selected to be more likely to reach the blastocyst stage has the capacity to successfully implant into the uterus.

AQ. The method of statement AP wherein the one or more human embryos with the capacity to successfully implant into the uterus has the capacity to go through gestation.

AR. The method of statement AQ wherein the one or more human embryos with the capacity to go through gestation has the capacity to be born live.

AS. A method for selecting one or more human in vitro fertilized embryos that is not likely to reach the usable blastocyst stage comprising: culturing one or more human embryos in vitro under conditions sufficient for embryo development; time lapse imaging said one or more human embryos; determining whether an embryo is of good or poor quality by morphological assessment; measuring cellular parameters comprising: (a) the time interval between mitosis 1 and mitosis 2; and (b) the time interval between mitosis 2 and mitosis; 3 and selecting an embryo that is not likely to reach the usable blastocyst stage when: 1. the morphological assessment determines the embryo to be of poor quality; or 2. the morphological assessment determines the embryo to be of good quality but the time interval between mitosis 1 and mitosis 2 is less than about 9.33 hours or more than about 11.45 hours or the interval between mitosis 2 and mitosis 3 is more than about 1.73 hours.

AT. The method of statement AS wherein the morphological assessment is done before, concurrently with or after the measurement of the cellular parameters.

AU. The method of statement AS wherein the morphological assessment is done at day 3 post insemination.

AV. The method of statement AU wherein the morphological assessment includes determining the number of cells, determining the level of fragmentation and/or determining the symmetry of the blastomeres.

AW. The method of statement AV wherein the morphological assessment determines an embryo is of poor quality when the embryo has less than 6 or more than 10 cells, has more than 10% fragmentation and has asymmetrical blastomeres.

AX. The method of statement AS wherein the measuring of the cellular parameters is automated.

AY. The method of statement AS wherein said one or more human embryos are placed in a culture dish prior to culturing under conditions sufficient for embryo development.

AZ. A method for sequentially analyzing a human in vitro fertilized embryo to select a human embryo that is likely to reach the blastocyst stage or deselect an embryo that is not likely to reach the usable blastocyst stage comprising: culturing the embryo in vitro under conditions sufficient for embryo development; determining whether the embryo is of good or poor quality by morphological assessment; time lapse imaging the embryo for the duration of at least one mitotic cell cycle when the embryo when the morphological assessment determines the embryo is of good quality; and selecting a human embryo that is likely to reach the blastocyst stage when the morphological assessment determines the embryo is of good quality and the time interval between mitosis 1 and mitosis 2 is about 9.33-11.45 hours and the time interval between mitosis 2 and mitosis 3 is about 0-1.73 hours; or deselecting a human embryo that is not likely to reach the blastocyst stage when the morphological assessment determines the embryo is of poor quality or the morphological assessment determines the embryo to be of good quality but the time interval between mitosis 1 and mitosis 2 is less than about 9.33 hours and more than about 11.45 hours or the time interval between mitosis 2 and mitosis 3 is more than about 1.73 hours.

BA. The method of statement AZ wherein the morphological assessment is done at day 3 post insemination.

BB. The method of statement BA wherein the morphological assessment includes determining the number of cells, determining the level of fragmentation and determining the symmetry of the blastomeres.

BC. The method of statement BB wherein the morphological assessment determines an embryo is of good quality when the embryo has 6-10 cells, has less than 10% fragmentation and/or has symmetrical blastomeres.

BD. The method of statement BC wherein the morphological assessment determines an embryo is of poor quality when the embryo has less than 6 or more than 10 cells, has more than 10% fragmentation and/or has asymmetrical blastomeres.

BE. The method of statement AZ further comprising implanting the human embryo selected to be more likely to reach the blastocyst stage into a female subject.

BF. The method of statement AZ further comprising freezing the human embryo selected to be more likely to reach the blastocyst stage.

BG. The method of statement AZ wherein said human embryo is placed in a culture dish prior to culturing under conditions sufficient for embryo development.

BH. The method of statement AZ wherein the human embryo selected to be more likely to reach the blastocyst stage has the capacity to successfully implant into the uterus.

BI. The method of statement BH wherein the human embryo with the capacity to successfully implant into the uterus has the capacity to go through gestation.

BJ. The method of statement BI wherein the human embryo with the capacity to go through gestation has the capacity to be born live.

## Claims

1. A method for selecting one or more human in vitro fertilized embryos that is likely to reach the blastocyst stage comprising:

   culturing one or more human embryos in vitro under conditions sufficient for embryo development;
   time lapse imaging said one or more human embryos;
   determining whether an embryo is of good or poor quality by a morphological assessment;
   measuring cellular parameters comprising:

   (a) the time interval between mitosis 1 and mitosis 2; and
   (b) the time interval between mitosis 2 and mitosis 3 and

   selecting an embryo that is likely to reach the blastocyst stage when:

   the morphological assessment determines the embryo is of good quality and the time interval between mitosis 1 and mitosis 2 is between 9.33-11.45 hours; and
   the time interval between mitosis 2 and mitosis 3 is between 0-1.73 hours,

   wherein the morphological assessment is done concurrently with the measurement of the cellular parameters wherein the morphological assessment includes determining the level of fragmentation and/or determining the symmetry of the blastomeres.

**2.** The method of claim 1 further comprising freezing the human embryo selected to be more likely to reach the blastocyst stage.

**3.** The method of claim 1 wherein the measuring of the cellular parameters is automated.

**4.** The method of claim 1 wherein said one or more human embryos are placed in a culture dish prior to culturing under conditions sufficient for embryo development.

**Patentansprüche**

**1.** Verfahren zur Auswahl eines oder mehrerer menschlicher in vitro befruchteter Embryonen, der wahrscheinlich das Blastozystenstadium erreichen wird, umfassend:

Kultivieren eines oder mehrerer menschlicher Embryonen in vitro unter Bedingungen, die für die Embryonenentwicklung ausreichend sind;
Zeitraffer-Aufnehmen des einen oder der mehreren menschlichen Embryonen;
Bestimmen, ob ein Embryo von guter oder schlechter Qualität ist, durch eine morphologische Beurteilung;
Messen von zellulären Parametern umfassend:

(a) das Zeitintervall zwischen Mitose 1 und Mitose 2; und
(b) das Zeitintervall zwischen Mitose 2 und Mitose 3 und

Auswählen eines Embryos, der wahrscheinlich das Blastozystenstadium erreichen wird, wenn:

die morphologische Beurteilung bestimmt, dass der Embryo von guter Qualität ist, und das Zeitintervall zwischen Mitose 1 und Mitose 2 zwischen 9,33-11,45 Stunden liegt; und
das Zeitintervall zwischen Mitose 2 und Mitose 3 zwischen 0-1,73 Stunden liegt,
wobei die morphologische Beurteilung zeitgleich mit der Messung der zellulären Parameter erfolgt, wobei die morphologische Beurteilung Bestimmen des Niveaus der Fragmentierung und/oder Bestimmen der Symmetrie der Blastomere aufweist.

**2.** Verfahren nach Anspruch 1, weiter umfassend Einfrieren des menschlichen Embryos, der so ausgewählt wird, dass er das Blastozystenstadium wahrscheinlicher erreichen wird.

**3.** Verfahren nach Anspruch 1, wobei das Messen der zellulären Parameter automatisiert ist.

**4.** Verfahren nach Anspruch 1, wobei der eine oder die mehreren menschlichen Embryonen vor dem Kultivieren unter Bedingungen, die für die Embryonenentwicklung ausreichend sind, in einer Petrischale platziert werden.

**Revendications**

**1.** Procédé de sélection d'un ou plusieurs embryons humains fertilisés *in vitro* qui sont susceptibles d'atteindre le stade de blastocyste comprenant :

la culture d'un ou plusieurs embryons humains *in vitro* dans des conditions suffisantes pour le développement des embryons ;
l'imagerie chronocinématographique desdits un ou plusieurs embryons humains ;
le fait de déterminer si un embryon est de bonne ou de mauvaise qualité par une évaluation morphologique ;
la mesure des paramètres cellulaires comprenant :

(a) l'intervalle de temps entre la mitose 1 et la mitose 2 ; et
(b) l'intervalle de temps entre la mitose 2 et la mitose 3 et

la sélection d'un embryon qui est susceptible d'atteindre le stade de blastocyste lorsque :

l'évaluation morphologique détermine que l'embryon est de bonne qualité et l'intervalle de temps entre la mitose 1 et la mitose 2 est entre 9,33-11,45 heures ; et

l'intervalle de temps entre la mitose 2 et la mitose 3 est entre 0-1,73 heures,

dans lequel l'évaluation morphologique est effectuée conjointement avec la mesure des paramètres cellulaires dans lequel l'évaluation morphologique inclut la détermination du niveau de fragmentation et/ou la détermination de la symétrie des blastomères.

2.  Procédé selon la revendication 1 comprenant en outre la congélation de l'embryon humain sélectionné comme étant plus susceptible d'atteindre le stade de blastocyste.

3.  Procédé selon la revendication 1 dans lequel la mesure des paramètres cellulaires est automatisée.

4.  Procédé selon la revendication 1 dans lequel lesdits un ou plusieurs embryons humains sont placés dans une boite de culture avant la culture dans des conditions suffisantes pour le développement des embryons.

Figure 1

Figure 2

| P2: 2nd Mitosis | P3: 3rd Mitosis |
|---|---|
| 9.33 to 11.45 Hours | 0 – 1.73 Hours |

Figure 3

| Node | Node Size | Number of Blasts | Number of Arrested |
|------|-----------|------------------|--------------------|
| 1 | 292 | 68 | 224 |
| 2 | 218 | 66 | 152 |
| 3 | 74 | 2 | 72 |
| 4 | 63 | 5 | 58 |
| 5 | 155 | 61 | 94 |
| 6 | 91 | 47 | 44 |
| 7 | 64 | 14 | 50 |
| 8 | 66 | 39 | 27 |
| 9 | 25 | 8 | 17 |

Figure 4

| Node | Node Size | Number of Blasts | Number of Arrested |
|------|-----------|------------------|--------------------|
| 1 | 292 | 68 | 224 |
| 2 | 218 | 66 | 152 |
| 3 | 74 | 2 | 72 |
| 4 | 63 | 5 | 58 |
| 5 | 155 | 61 | 94 |
| 6 | 91 | 47 | 44 |
| 7 | 64 | 14 | 50 |
| 8 | 84 | 46 | 38 |
| 9 | 7 | 1 | 6 |

Figure 8

Figure 6

Figure 7

Eeva Parameters Validated with Time-Lapse

Figure 8

Figure 9

| Eeva vs. Manual | % Agreement | 95 % CI |
|---|---|---|
| Overall % Agreement | 91.0 | (86.0, 94.3) |

Figure 10

Figure 11

Figure 12

Figure 13

**All Embryos (n=755)**

A

■ Using morphology only

Using morphology plus Eeva

**Good Morphology Embryos (n=235)**

B

■ Using morphology only

Using morphology plus Eeva

Figure 14

```
                              ┌─────────────────┐
                              │      Day 3      │
                              │   Morphology    │
                              └─────────────────┘
                         │                           │
                         ▼                           ▼
              ┌─────────────────┐            ┌─────────────────┐
              │    Good/Fair    │            │      Poor       │
              │     (A,B,C)     │            │       (D)       │
              └─────────────────┘            └─────────────────┘
           │                     │                   │
           ▼                     ▼                   ▼
   ┌──────────────┐      ┌──────────────┐    ┌──────────────┐
   │   Eeva High  │      │   Eeva Low   │    │   Eeva N/A   │
   └──────────────┘      └──────────────┘    └──────────────┘
           │                     │
           ▼                     ▼
   ┌──────────────┐      ┌──────────────┐
   │  Top Choices │      │  2nd Choices │
   └──────────────┘      └──────────────┘
```

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2007144001 A
- US 7963906 B
- US 6610543 B
- US 6130086 A
- US 5837543 A
- US 5882928 A
- US 6281013 B
- US 6777233 B
- US 7037892 B
- US 7029913 B
- US 5843780 A
- US 6200806 B
- US 20090047263 A
- US 20090068742 A
- WO 2012047678 A
- US 2010041090 A
- US 20120309043 A
- US 20130102837 A
- US 20110183367 A
- US 201101656909 A
- US 20110111447 A
- WO 2012163363 A
- WO 2013004239 A
- WO 2013029625 A
- US 20120140056 A

**Non-patent literature cited in the description**

- **TAFT, R.E.** *Theriogenology,* 2008, vol. 69 (1), 10-16
- **BELL, C. E. et al.** *Mol. Hum. Reprod.,* 2008, vol. 14, 691-701
- **BRAUDE, P. et al.** *Nature,* 1988, vol. 332, 459-461
- **HAMATANI, T. et al.** *Proc. Natl. Acad. Sci.,* 2004, vol. 101, 10326-10331
- **DOBSON, T. et al.** *Human Molecular Genetics,* 2004, vol. 13 (14), 1461-1470
- **RIENZI, L. et al.** *Reprod. Biomed. Online,* 2005, vol. 10, 669-681
- **ALIKANI, M. et al.** *Mol. Hum. Reprod.,* 2005, vol. 11, 335-344
- **KELTZ, M. D. et al.** *Fertil. Steril.,* 2006, vol. 86, 321-324
- **FRENCH, D. B. et al.** *Fertil. Steril.,* 2009
- **ZEMICKA-GOETZ, M.** *Development,* 2002, vol. 129, 815-829
- **WANG, Q. et al.** *Dev Cell,* 2004, vol. 6, 133-144
- **ZEMICKA-GOETZ, M.** *Curr. Opin. Genet. Dev.,* 2006, vol. 16, 406-412
- **MTANGO, N. R. et al.** *Int. Rev. Cell. Mol. Biol.,* 2008, vol. 268, 223-290
- **RIJINDERS PM ; JANSEN CAM.** *Hum Reprod,* 1998, vol. 13, 2869-73
- **MILKI AA et al.** *Fertil Steril,* 2002, vol. 77, 1191-5
- **MILKI A et al.** *Fertil Steril,* 2000, vol. 73, 126-9
- **FRAGOULI E.** *Fertil Steril,* 21 June 2009
- **EL-TOUKHY T et al.** *Hum Reprod,* 2009, vol. 6, 20
- **VANNESTE E et al.** *Nat Med,* 2009, vol. 15, 577-83
- **MANIPALVIRATN S et al.** *Fertil Steril,* 2009, vol. 91, 305-15
- **MASTENBROEK S et al.** *N Engl J Med.,* 2007, vol. 357, 9-17
- **NAGY et al.** *Human Reproduction,* 1994, vol. 9 (9), 1743-1748
- **PAYNE et al.** *Human Reproduction,* 1997, vol. 12, 532-541
- **FENWICK et al.** *Human Reproduction,* 2002, vol. 17, 407-412
- **LUNDIN et al.** *Human Reproduction,* 2001, vol. 16, 2652-2657
- **TAFT.** *Theriogenology,* 2008, vol. 69 (1), 10-16
- **LEMMEN et al.** *Reproductive BioMedicine Online,* 2008, vol. 17 (3), 385-391
- **CONNIE C WONG et al.** Non-invasive imaging of human embryos before embryonic genome activation predicts development to the blastocyst stage. *Nature Biotechnology,* 01 October 2010, vol. 28, 115-1121
- **MESEGUER et al.** The use of morphokinetics as a predictor of embryo implantation. *Human Reproduction,* 09 August 2011, vol. 26 (10), 2658-2671
- **KATARI et al.** *Hum Mol Genet.,* 2009, vol. 18 (20), 3769-78
- **SEPULVEDA et al.** *Fertil Steril.,* 2009, vol. 91 (5), 1765-70

- *The Instanbul Consensus Workshop on Embryo Assessment: Proceedings of an expert meeting,* 2011, vol. 22
- **WONG CC ; LOEWKE KE ; BOSSERT NL ; BEHR B ; DE JONGE CJ ; BAER TM ; REIJO PERA RA.** Non-Invasive Imaging of Human Embryos Before Embryonic Genome Activation Predicts Development to the Blastocyst Stage. *Nat Biotechnol.,* October 2010, vol. 28 (10), 1115-21
- **ALPHA E.** The Istanbul consensus workshop on embryo assessment: proceedings of an expert meeting. *Hum Reprod,* 2011, vol. 26, 1270-1283
- **ASSIDI M ; MONTAG M ; VEN K ; SIRARD MA.** Biomarkers of human oocyte developmental competence expressed in cumulus cells before ICSI: a preliminary study. *J Assist Reprod Genet,* 2011, vol. 28, 173-188
- **BARLOW P ; PUISSANT F ; ZWALMEN P ; VANDROMME J ; TRIGAUX P ; LEROY F.** In vitro fertilization, development, and implantation after exposure of mature mouse oocytes to visible light. *Mol Reprod Dev,* 1992, vol. 33, 297-302
- **BAXTER BENDUS AE ; MAYER JF ; SHIPLEY SK ; CATHERINO WH.** Interobserver and intraobserver variation in day 3 embryo grading. *Fertil Steril,* 2006, vol. 86, 1608-1615
- **BLAKE DA ; FARQUHAR CM ; JOHNSON N ; PROCTOR M.** Cleavage stage versus blastocyst stage embryo transfer in assisted conception. *Cochrane Database Syst Rev,* 2007, CD002118
- **CHEN AA ; TAN L ; SURAJ V ; REIJO PERA R ; SHEN S.** Biomarkers identified with time-lapse imaging: discovery, validation, and practical application. *Fertil. Steril,* 2013, vol. 99, 1035-43
- **COSKUN S ; HOLLANDERS J ; AL-HASSAN S ; AL-SUFYAN H ; AL-MAYMAN H ; JAROUDI K.** Day 5 versus day 3 embryo transfer: a controlled randomized trial. *Hum Reprod,* 2000, vol. 15, 1947-1952
- **CRUZ M ; GADEA B ; GARRIDO N ; PEDERSEN KS ; MARTINEZ M ; PEREZ-CANO I ; MUNOZ M ; MESEGUER M.** Embryo quality, blastocyst and ongoing pregnancy rates in oocyte donation patients whose embryos were monitored by time-lapse imaging. *J Assist Reprod Genet,* 2011
- **CRUZ, M ; GARRIDO N ; HERRERO J ; PEREZ-CANO, I ; MUNOZ M ; MESEGUER M.** Timing of cell division in human cleavage-stage embryos is linked with blastocyst formation and quality. *Reprod. Biomed. Online,* 2012, vol. 25, 371-81
- **DE PLACIDO G ; WILDING M ; STRINA I ; ALVIGGI E ; ALVIGGI C ; MOLLO A ; VARICCHIO MT ; TOLINO A ; SCHIATTARELLA C ; DALE B.** High outcome predictability after IVF using a combined score for zygote and embryo morphology and growth rate. *Hum Reprod,* 2002, vol. 17, 2402-2409
- **DESAI NN ; GOLDSTEIN J ; ROWLAND DY ; GOLDFARB JM.** Morphological evaluations of human embryos and derivation of an embryo quality scornig system specific for day 3 embryos: a preliminary study. *Human Reprod 2000,* 2000, vol. 15, 2190-2196
- **DIAMOND MP ; WILLMAN S ; CHENETTE P ; CEDARS MI.** The clinical need for a method of identification of embryos destined to become a blastocyst in assisted reproductive technology cycles. *J Assist Reprod Genet,* 2012, vol. 29, 391-396
- **FENWICK J ; PLATTEAU P ; MURDOCH AP ; HERBERT M.** Time from insemination to first cleavage predicts developmental competence of human pre-implantation embryos in vitro. *Hum Reprod,* 2002, vol. 17, 407-412
- **FILHO ES ; NOBLE JA ; POLI M ; GRIFFITHS T ; EMERSON G ; WELLS D.** A method for semi-automatic grading of human blastocyst microscope images. *Hum Reprod,* 2012
- **FILHO ES ; NOBLE JA ; WELLS D.** A review on automatic analysis of human embryo microscope images. *Open Biomed Eng J,* 2010, vol. 4, 170-177
- **FRAGOULI E ; WELLS D.** Aneuploidy screening for embryo selection. *Semin Reprod Med,* 2012, vol. 30, 289-301
- **GARDNER DK ; LANE M ; STEVENS J ; SCHLENKER T ; SCHOOLCRAFT WB.** Blastocyst score affects implantation and pregnancy outcome: towards a single blastocyst transfer. *Fertil Steril,* 2000, vol. 73, 1155-1158
- **GELBAYA TA ; TSOUMPOU I ; NARDO LG.** The likelihood of live birth and multiple birth after single versus double embryo transfer at the cleavage stage: a systematic review and meta-analysis. *Fertil Steril,* 2010, vol. 94, 936-945
- **GONZALES DS ; PINHEIRO JC ; BAVISTER BD.** Prediction of the developmental potential of hamster embryos in vitro by precise timing of the third cell cycle. *J Reprod Fertil,* 1995, vol. 105, 1-8
- **GRISART B ; MASSIP A ; DESSY F.** Cinematographic analysis of bovine embryo development in serum-free oviduct-conditioned medium. *J Reprod Fertil,* 1994, vol. 101, 257-264
- **GUERIF F ; LEMSEFFER M ; LEGER J ; BIDAULT R ; CADORET V ; CHAVEZ C ; GASNIER O ; SAUSSEREAU MH ; ROYERE D.** Does early morphology provide additional selection power to blastocyst selection for transfer?. *Reprod Biomed Online,* 2010, vol. 21, 510-519
- **HARDARSON T ; HANSON C ; SJOGREN A ; LUNDIN K.** Human embryos with unevenly sized blastomeres have lower pregnancy and implantation rates: indications for aneuploidy and multinucleation. *Hum Reprod,* 2001, vol. 16, 313-318

- **HASHIMOTO S ; KATO N ; SAEKI K ; MORIMOTO Y.** Selection of high-potential embryos by culture in poly(dimethylsiloxane) microwells and time-lapse imaging. *Fertil Steril,* 2012, vol. 97, 332-337
- **HORSTHEMKE B ; LUDWIG M.** Assisted reproduction: the epigenetic perspective. *Hum Reprod Update,* 2005, vol. 11, 473-482
- **KALLEN B ; FINNSTROM O ; LINDAM A ; NILSSON E ; NYGREN KG ; OTTERBLAD OLAUSSON P.** Trends in delivery and neonatal outcome after in vitro fertilization in Sweden: data for 25 years. *Hum Reprod,* 2010, vol. 25, 1026-1034
- **KALRA SK ; RATCLIFFE SJ ; BARNHART KT ; COUTIFARIS C.** Extended Embryo Culture and an Increased Risk of Preterm Delivery. *Obstetrics & Gynecology,* 2012, vol. 120, 69-75
- **KIRKEGAARD K ; AGERHOLM IE ; INGERSLEV HJ.** Time-lapse monitoring as a tool for clinical embryo assessment. *Hum Reprod,* 2012, vol. 27, 1277-1285
- **KIRKEGAARD K ; HINDKJAER JJ ; GRONDAHL ML ; KESMODEL US ; INGERSLEV HJ.** A randomized clinical trial comparing embryo culture in a conventional incubator with a time-lapse incubator. *J Assist Reprod Genet,* 2012
- **LEMMEN JG ; AGERHOLM I ; ZIEBE S.** Kinetic markers of human embryo quality using time-lapse recordings of IVF/ICSI-fertilized oocytes. *Reprod Biomed Online,* 2008, vol. 17, 385-391
- **LUNDIN K ; BERGH C ; HARDARSON T.** Early embryo cleavage is a strong indicator of embryo quality in human IVF. *Hum Reprod,* 2001, vol. 16, 2652-2657
- **MACHITER R ; RACOWSKY C.** Morphological systems of human embryo assesment and clinical evidence. *Reproductive Bio Medicine Online,* 2013, vol. 26, 210-221
- **MANIPALVIRATN S ; DECHERNEY A ; SEGARS J.** Imprinting disorders and assisted reproductive technology. *Fertil Steril,* 2009, vol. 91, 305-315
- **MESEGUER M ; HERRERO J ; TEJERA A ; HILLIGSOE KM ; RAMSING NB ; REMOHI J.** The use of morphokinetics as a predictor of embryo implantation. *Hum Reprod,* 2011, vol. 26, 2658-71
- **MILKI AA ; HINCKLEY MD ; BEHR B.** Comparison of blastocyst transfer to day 3 transfer with assisted hatching in the older patient. *Fertil Steril,* 2002, vol. 78, 1244-1247
- **MILKI AA ; JUN SH ; HINCKLEY MD ; BEHR B ; GIUDICE LC ; WESTPHAL LM.** Incidence of monozygotic twinning with blastocyst transfer compared to cleavage-stage transfer. *Fertil Steril,* 2003, vol. 79, 503-506
- **MIO Y ; MAEDA K.** Time-lapse cinematography of dynamic changes occurring during in vitro development of human embryos. *Am J Obstet Gynecol,* 2008, vol. 199, 660 e661-665
- **MONTAG M ; LIEBENTHRON J ; KOSTER M.** Which morphological scoring system is relevant in human embryo development?. *Placenta,* 2011, vol. 32, S252-S256
- **NAKAHARA T ; IWASE A ; GOTO M ; HARATA T ; SUZUKI M ; IENAGA M ; KOBAYASHI H ; TAKIKAWA S ; MANABE S ; KIKKAWA F et al.** Evaluation of the safety of time-lapse observations for human embryos. *J Assist Reprod Genet,* 2010, vol. 27, 93-96
- **NEL-THEMAAT L ; NAGY ZP.** A review of the promises and pitfalls of oocyte and embryo metabolomics. *Placenta,* 2011, vol. 32 (3), S257-263
- **NIEMITZ EL ; FEINBERG AP.** Epigenetics and assisted reproductive technology: a call for investigation. *Am J Hum Genet,* 2004, vol. 74, 599-609
- **PAPANIKOLAOU EG ; CAMUS M ; KOLIBIANAKIS EM ; VAN LANDUYT L ; VAN STEIRTEGHEM A ; DEVROEY P.** In vitro fertilization with single blastocyst-stage versus single cleavage-stage embryos. *N Engl J Med,* 2006, vol. 354, 1139-1146
- **PAPANIKOLAOU EG ; D'HAESELEER E ; VERHEYEN G ; VAN DE VELDE H ; CAMUS M ; VAN STEIRTEGHEM A ; DEVROEY P ; TOURNAYE H.** Live birth rate is significantly higher after blastocyst transfer than after cleavage-stage embryo transfer when at least four embryos are available on day 3 of embryo culture. A randomized prospective study. *Hum Reprod,* 2005, vol. 20, 3198-3203
- **PATEMOT G ; DEROE J ; DEBROCK S ; D'HOOGHE TM ; SPIESSENS C.** Intra-and Inter-observer analysis in the morphligical assessment of early-stage embryos. *Reproductive biology and endocrinology: RB&E,* 2009, vol. 7, 105
- **PAYNE D ; FLAHERTY SP ; BARRY MF ; MATTHEWS CD.** Preliminary observations on polar body extrusion and pronuclear formation in human oocytes using time-lapse video cinematography. *Hum Reprod,* 1997, vol. 12, 532-541
- **PRIBENSZKY C ; LOSONCZI E ; MOLNAR M ; LANG Z ; MATYAS S ; RAJCZY K ; MOLNAR K ; KOVACS P ; NAGY P ; CONCEICAO J et al.** Prediction of in-vitro developmental competence of early cleavage-stage mouse embryos with compact time-lapse equipment. *Reprod Biomed Online,* 2010, vol. 20, 371-379
- **RACOWSKY C ; VERNON M ; MAYER J ; BALL GD ; BEHR B ; POMEROY KO ; WININGER D ; GIBBONS W ; CONAGHAN J ; STERN JE.** Standardization of grading embryo morphology. *Fertil Steril,* 2010, vol. 94, 1152-1153

- **RIJNDERS PM ; JANSEN CA.** Influence of group culture and culture volume on the formation of human blastocysts: a prospective randomized study. *Hum Reprod,* 1999, vol. 14, 2333-2337
- **SCOTT R ; TAO X ; TAYLOR D ; FERRY KM ; TREFF NR.** A prospective randomized controlled trial demonstrating significantly increased clinical pregnancy rates following 24 chromosome aneuploidy screening: biopsy and analysis on day 5 with fresh transfer. *Fertil Steril,* 2010, vol. 94, S2
- **SCOTT RT, JR. ; FERRY K ; SU J ; TAO X ; SCOTT K ; TREFF NR.** Comprehensive chromosome screening is highly predictive of the reproductive potential of human embryos: a prospective, blinded, nonselection study. *Fertil Steril,* 2012, vol. 97, 870-875
- **SELI E ; ROBERT C ; SIRARD MA.** OMICS in assisted reproduction: possibilities and pitfalls. *Mol Hum Reprod,* 2010, vol. 16, 513-530
- **SELI E ; VERGOUW CG ; MORITA H ; BOTROS L ; ROOS P ; LAMBALK CB ; YAMASHITA N ; KATO O ; SAKKAS D.** Noninvasive metabolomic profiling as an adjunct to morphology for noninvasive embryo assessment in women undergoing single embryo transfer. *Fertil Steril,* 2010, vol. 94, 535-542
- **SUGIMURA S ; AKAI T ; SOMFAI T ; HIRAYAMA M ; AIKAWA Y ; OHTAKE M ; HATTORI H ; KOBAYASHI S ; HASHIYADA Y ; KONISHI K et al.** Time-lapse cinematography-compatible polystyrene-based microwell culture system: a novel tool for tracking the development of individual bovine embryos. *Biol Reprod,* 2010, vol. 83, 970-978
- **SWANN K ; WINDSOR S ; CAMPBELL K ; ELGMATI K ; NOMIKOS M ; ZERNICKA-GOETZ M ; AMSON ; LAI FA ; THOMAS A ; GRAHAM C.** Phospholipase C-zeta-induced Ca2+ oscillations cause coincident cytoplasmic movements in human oocytes that failed to fertilize after intracytoplasmic sperm injection. *Fertil Steril,* 2012, vol. 97, 742-747
- **VAJTA G ; PEURA TT ; HOLM P ; PALDI A ; GREVE T ; TROUNSON AO ; CALLESEN H.** New method for culture of zona-included or zona-free embryos: the Well of the Well (WOW) system. *Mol Reprod Dev,* 2000, vol. 55, 256-264
- **VAN MONTFOORT AP ; DUMOULIN JC ; LAND JA ; COONEN E ; DERHAAG JG ; EVERS JL.** Elective single embryo transfer (eSET) policy in the first three IVF/ICSI treatment cycles. *Hum Reprod,* 2005, vol. 20, 433-436
- **VERGOUW CG ; KIESLINGER DC ; KOSTELIJK EH ; BOTROS LL ; SCHATS R ; HOMPES PG ; SAKKAS D ; LAMBALK CB.** Day 3 embryo selection by metabolomic profiling of culture medium with near-infrared spectroscopy as an adjunct to morphology: a randomized controlled trial. *Hum Reprod,* 2012
- **VERNON M ; STERN JE ; BALL GD ; WININGER D ; MAYER J ; RACOWSKY C.** Utility of the national embryo morphology data collection by the Society for Assisted Reproductive Technologies (SART): correlation between day-3 morphology grade and live-birth outcome. *Fertil Steril,* 2011, vol. 95, 2761-2763
- **WATHLET S ; ADRIAENSSENS T ; SEGERS I ; VERHEYEN G ; JANSSENS R ; COUCKE W ; DEVROEY P ; SMITZ J.** New candidate genes to predict pregnancy outcome in single embryo transfer cycles when using cumulus cell gene expression. *Fertil Steril,* 2012
- **WATHLET S ; ADRIAENSSENS T ; SEGERS I ; VERHEYEN G ; VAN DE VELDE H ; COUCKE W ; RON EL R, DEVROEY P ; SMITZ J.** Cumulus cell gene expression predicts better cleavage-stage embryo or blastocyst development and pregnancy for ICSI patients. *Hum Reprod,* 2011, vol. 26, 1035-1051
- **WONG C ; LOEWKE K ; BOSSERT N ; BEHR B ; DE JONGE C ; BAER T ; REIJO PERA R.** Non-invasive imaging of human embryos before embryonic genome activation predicts development to the blastocyst stage. *Nat Biotechnol,* 2010, vol. 28, 1115-1121
- **YANG Z ; LIU J ; COLLINS GS ; SALEM SA ; LIU X ; LYLE SS ; PECK AC ; SILLS ES ; SALEM RD.** Selection of single blastocysts for fresh transfer via standard morphology assessment alone and with array CGH for good prognosis IVF patients: results from a randomized pilot study. *Molecular cytogenetics,* 2012, vol. 5, 24